Europäisches Patentamt

European Patent Office

Office européen des brevets

(19)

(11) **EP 0 702 083 A2**

(12) **EUROPÄISCHE PATENTANMELDUNG**

(43) Veröffentlichungstag:
20.03.1996 Patentblatt 1996/12

(51) Int. Cl.⁶: **C12N 15/62**, C07K 14/045,
G01N 33/569, C12Q 1/70

(21) Anmeldenummer: 95110981.8

(22) Anmeldetag: 13.07.1995

(84) Benannte Vertragsstaaten:
AT CH DE ES FR GB IT LI

(30) Priorität: 26.07.1994 DE 4426453
06.10.1994 DE 4435789

(71) Anmelder: BIOTEST AG
D-63303 Dreieich (DE)

(72) Erfinder:
• Vornhagen, Rolf, Dr.
D-63225 Langen (DE)
• Hinderer, Walter, Dr.
D-63110 Rodgau (DE)
• Sonneborn, Hans-H., Dr.
D-63150 Heusenstamm (DE)
• Plachter, Bodo, Dr.
D-91083 Baiersdorf (DE)

• Jahn, Gerhard, Prof.Dr.
D-72108 Rottenburg (DE)

(74) Vertreter: Keller, Günter, Dr. et al
Lederer, Keller & Riederer
Patentanwälte
Prinzregentenstrasse 16
D-80538 München (DE)

Bemerkungen:
Ein Antrag gemäss Regel 88 EPÜ auf Berichtigung
des ursprünglich eingereichten Sequenzprotokolls
liegt vor. Über diesen Antrag wird im Laufe des
Verfahrens von der Prüfungsabteilung eine
Entscheidung getroffen werden (Richtlinien für die
Prüfung im EPA, A-V, 3.).

(54) **Polypeptide und Fusionsproteine bestehend aus dem UL 57 Leserahmen bzw. dem C-terminalen Bereich des Tegumentproteins pp150 aus HCMV, entsprechende Oligonucleotide und Nachweis reagentien**

(57) Offenbart werden diagnostisch relevante Polypeptide bzw. Fusionsproteine umfassend eine vom Cytomegalievirus stammende Aminosäuresequenz, die einem Bereich des Major-DNA-binding proteins oder des C-terminalen Bereichs des Tegumentproteins pp150 fusioniert mit wenigstens einem weiteren Fragment aus einem anderen antigenen Protein des Cytomegalievirus entspricht. Das Major-DNA-binding-Protein wird durch den Leserahmen UL 57 codiert. Die erfindungsgemäßen Polypeptide bzw. Fusionsproteine können in vorteilhafter Weise in diagnostischen Tests und Verfahren zum Nachweis von IgM-Antikörper gegen Cytomegalievirus verwendet werden.

EP 0 702 083 A2

**Beschreibung**

Infektionen des Menschen mit dem humanen Cytomegalievirus (HCMV) sind weit verbreitet und verlaufen in der Regel symptomlos während der Kindheit. HCMV-spezifische IgG-Antikörper als Marker für eine abgelaufene/latente Infektion lassen sich je nach getesteter Population bei 50-90% der Erwachsenen feststellen. Congenitale Infektion sowie akute Infektionen bei Immunsupprimierten wie Transplantationspatienten und HIV-Infizierten können zu einer lebensbedrohenden Krankheit führen.

Um eine frühzeitige und effektive antivirale Therapie sicherzustellen, ist es von großem Interesse festzustellen, ob eine akute Infektion vorliegt. Zur Diagnose einer akuten HCMV-Infektion können verschiedene Methoden zur Anwendung kommen. Insbesondere bei Transplantationspatienten, bei denen eine ständige, begleitende Diagnostik möglich ist, haben sich in den letzten Jahren der pp65-spezifische Antigenemietest sowie die PCR aufgrund ihrer überlegenen Sensitivität weitgehend durchgesetzt. Im Routinelabor erfolgt die Diagnose einer akuten Infektion, z. B. im Rahmen der viralen Differentialdiagnose bzw. während der Schwangerschaftsvorsorge, in der Regel durch den Nachweis des Erregers in der Zellkultur sowie serologisch durch den Nachweis spezifischer Antikörper, insbesondere im ELISA. Als spezifische Antigene für den Antikörpernachweis werden z. Z. ausschließlich mehr oder weniger gereinigte, schlecht charakterisierte Lysate HCMV-infizierter Zellkulturen verwendet. Dies führt insbesondere beim IgM-Nachweis zu Spezifitäts- bzw. Sensitivitätsproblemen. Es existieren zahlreiche Publikationen, die die diagnostische Relevanz verschiedener rekombinanter Antigene bzw. chemisch synthetisierter Peptide behandeln. Die bisher vorliegenden Ergebnisse sind in M.P. Landini: "Antibody Responses to Human Cytomegalovirus Proteins", Reviews in Medical Virology **2**, 63-72 (1992) zusammengefaßt.

Aufgabe der vorliegenden Erfindung ist es daher, Polypeptide und/oder Fusionsproteine bereitzustellen, die für die Diagnostik vorteilhaft sind, sowie Testkits, die diese enthalten und Verfahren zum Nachweis von akuten Infektionen des HCMV.

Unter dem Begriff Polypeptide werden im Rahmen der vorliegenden Anmeldung aus $\alpha$-Aminosäuren bestehende Biomoleküle verstanden, die mehr als 10 Aminosäuren aufweisen. Im Hinblick auf die obere Grenze der Polypeptide ist zu bemerken, daß es sich nicht um komplette Proteine oder nahezu komplette Proteine handelt. Wenn die erfindungsgemäßen Polypeptide in Form von Fusionsproteinen vorliegen, können diese durchaus auch mehr als 100 Aminosäuren aufweisen. Die erfindungsgemäßen Polypeptide können, wenn sie als Fusionsproteine vorliegen, durchaus eine Länge von etwa 300 Aminosäuren aufweisen. Wesentlich ist aber, daß die erfindungsgemäßen Polypeptide nur bestimmte Bereiche aus den Proteinen von Cytomegalievirus aufweisen.

Für diagnostische Tests sind zwei Aspekte von besonderer Bedeutung. Einerseits müssen die Antigene hochspezifisch sein, d.h. in dem diagnostischen Test muß klar erkennbar sein, daß es sich um den gesuchten Erreger handelt und nicht um einen anderen Erreger. Andererseits muß der diagnostische Test hochsensitiv sein, d.h. es muß möglichst zuverlässig jede Infektion mit dem gesuchten Erreger nachgewiesen werden können, auch wenn nur verhältnismäßig wenige Antikörper in der zu untersuchenden Probe vorliegen. Falsch positive Ergebnisse sollten möglichst nicht auftreten.

Die einzelnen durch das humane Cytomegalievirus codierten Proteine werden in der Regel durch den Leserahmen, durch den sie codiert sind, gekennzeichnet. Landini (a.a.O.) hat beispielsweise die genomische Lokalisation der verschiedenen bereits bekannten Proteine des HCM-Virus angegeben. Der Leserahmen kann daher der Charakterisierung des Proteins dienen.

Der Leserahmen UL 57 des humanen Cytomegalievirus codiert für das sogenannte Major-DNA-binding protein, das ein Molekulargewicht von etwa 130 bis 133 kD aufweist. Anders et al. haben das Protein und den Leserahmen in J.Virology, **62**, S. 1364-1372 (1988) charakterisiert. Interessanterweise weist der Leserahmen UL 57 eine signifikante Homologie zu den entsprechenden Leserahmen anderer Herpesviren auf, insbesondere zu den Viren HHV-6 und in geringerem Ausmaß zu EBV (Epstein-Barr-Virus). Aufgrund dieser Homologie zu den Leserahmen anderer Viren wäre das durch UL 57 codierte Major-DNA-binding protein eigentlich nicht für die Diagnostik prädestiniert, da die Gefahr von Kreuzreaktionen besteht.

Im Rahmen der vorliegenden Erfindung wurde aber überraschend festgestellt, daß ein Bereich aus dem Leserahmen UL 57 in besonders vorteilhafter Weise für die Diagnostik verwendet werden kann, da Polypeptide sowie Fusionsproteine, die diese Aminosäuresequenz aufweisen, in diagnostischen Tests vorteilhafte Ergebnisse ermöglichen. Im Rahmen der vorliegenden Erfindung wurden drei Fragmente aus dem Leserahmen UL 57 bereitgestellt, wobei die beiden Fragmente UL 57/1 und UL 57/3 mittels Polymerase Kettenreaktion amplifiziert werden konnten und das Fragment UL 57/2 wurde mittels chemischer DNA-Synthese bereitgestellt. Diese DNA-Fragmente wurden in einen Expressionsvektor (pGEX-3) umkloniert, der die Expression der viralen Antigene in Form von Fusionsproteinen (mit der Glutathion-S-Transferase (GST)) ermöglicht. Die Fusionsantigene wurden gereinigt und in ELISA-Experimenten mit ausgewählten Seren getestet. Dabei ergab sich überraschenderweise, daß das aus 57 AS bestehende Antigenfragment UL 57/3 ein hervorragendes Antigen zum spezifischen und sensitiven Nachweis von IgM-Antikörpern während der akuten HCMV-Infektion ist. Die beiden anderen Fragmente erwiesen sich hingegen als diagnostisch nicht relevant. Da UL 57/3 aufgrund seiner geringen Größe nur schwer als Nichtfusionsprotein in E.coli exprimiert werden kann, erfolgte die Expression auch als autologes Fusionsprotein zusammen mit weiteren diagnostisch relevanten HCMV-Antigenfragmenten, die ebenfalls eine spezifische und sensitive IgM-Serodiagnostik gewährleisten. Beim autologen Fusionsprotein 52/3 57/3 erfolgte die Expression C-terminal mit den Aminosäuren 297-433 von p52. Beim autologen Fusionsprotein 52/3 57/3 150/7 wurden am C-terminalen Ende von 57/3 zusätzlich die 54 C-terminalen Aminosäuren von pp150 exprimiert. Außerdem wurde UL57/3 als Peptid chemisch synthetisiert.

Gegenstand der vorliegenden Erfindung sind daher Polypeptide umfassend eine vom Cytomegalievirus (HCMV) stammende Aminosäuresequenz, die aus dem Leserahmen UL 57 des HCM-Virus stammt, wobei dieser Leserahmen für das Major-DNA-binding protein codiert, die dadurch gekennzeichnet sind, daß sie eine Homologie von wenigstens 60 % zu der Aminosäuresequenz

```
Gly Val Pro Gly Gly Gly Ala Gly Gly Gly Gly Gly Arg Asp Val

Ser Gly Gly Pro Ser Asp Gly Leu Gly Gly Gly Arg Gly Gly Gly

Gly Gly Gly Asp Ser Gly Gly Met Met Gly Arg Gly Gly Arg Met

Leu Gly Ala Ser Val Asp Arg Thr Tyr Arg Leu Asn
```

aufweisen.

Bevorzugterweise haben die erfindungsgemäßen Polypeptide eine Homologie von wenigstens 80 % zu der oben dargestellten Aminosäuresequenz. In besonders bevorzugter Ausführungsform weisen die erfindungsgemäßen Polypeptide die oben dargestellte Aminosäuresequenz oder eine Teilsequenz davon auf. Üblicherweise haben die erfindungsgemäßen Polypeptide eine Länge von wenigstens 10 Aminosäuren; bevorzugt sind aber Polypeptide mit einer Länge von wenigstens 25 Aminosäuren und besonders bevorzugt sind Polypeptide mit einer Länge von wenigstens 40 Aminosäuren.

Die erfindungsgemäßen Peptide können durch an sich bekannte chemische Methoden (beispielsweise Festphasensynthese) synthetisiert werden, oder auch durch gentechnische Methoden hergestellt werden.

In der EPA 87.111726.3 wird ein strukturelles Phosphorprotein (pp150) des menschlichen Cytomegalievirus beschrieben, das sich für den Nachweis von spezifischen Antikörpern als bedeutsam erwiesen hat.

Gegen HCMV gerichtete IgG-Antikörper zeigen, daß eine Infektion mit dem humanen Cytomegalievirus irgendwann stattgefunden hat. Durch die Bestimmung des Titers der IgM-Antikörper gegen HCMV kann man dagegen feststellen, ob eine frische Infektion mit HCMV-Viren vorliegt oder nicht. Die Beantwortung dieser Frage ist insbesondere bei den erwähnten Risikopatienten sowie bei schwangeren Frauen von großer Bedeutung. Bei einem derartigen Test ist es von besonderer Bedeutung, daß der Nachweis hochspezifisch und hochsensitiv ist. Wenn bei dem Test eine zu hohe Zahl von positiven, aber nicht bestätigbaren Ergebnissen erhalten wird, hat der Test nur eine geringe Aussagekraft. Andererseits muß jedoch der Test auch mit hinreichender Sicherheit frische HCMV-Infektionen erkennen lassen, wobei aber die Zahl der falsch positiven Ergebnisse so weit wie möglich reduziert sein sollte.

Die DNA-Sequenz des Cytomegalievirus ist bereits bekannt. Chee et al. offenbaren in ihrer Veröffentlichung in Current Topics in Microbiology and Immunobiology, Vol. 154 (1990), S. 125-169, die Fundstellen der DNA-Sequenz und eine Analyse der kodierenden Sequenzen des Cytomegalievirus. Diese Literaturstelle wird hiermit durch Bezugnahme in den Offenbarungsgehalt der vorliegenden Anmeldung mit eingeschlossen.

Im Rahmen der vorliegenden Erfindung konnte festgestellt werden, daß der C-terminale Bereich des pp150-Tegumentproteins ein sehr sensitives Antigen zum Nachweis von IgM-Antikörpern ist. Im Rahmen der vorliegenden Erfindung handelt es sich hierbei um einen Bereich von 40 bis 60 Aminosäuren am C-Terminus des pp150-Proteins. Da die Aminosäuresequenz des pp150-Proteins bereits bekannt ist, handelt es sich hierbei um den Bereich der Aminosäuren 988 bis 1048. Besonders bevorzugt im Rahmen der vorliegenden Erfindung wird der Bereich des pp150-Tegumentproteins, der die Aminosäuren 994 bis 1048 umfaßt und dieser Bereich wird vorliegend bezeichnet als *pp150/7/2*.

Dieser C-terminale Bereich des pp150-Tegumentproteins wird erfindungsgemäß fusioniert an einen Bereich mit hoher IgM-Reaktivität, der von einem anderen Antigen des HCMV-Virus stammt. Andere Antigene sind beispielsweise die als pp65, pp71, pp28 oder gp116/58 bezeichneten Antigene. Als im Rahmen der vorliegenden Erfindung besonders geeignetes Antigen hat sich das Antigen p52 und insbesondere der C-terminale Bereich des Antigens p52 herausgestellt.

Im Rahmen der vorliegenden Erfindung wird also bevorzugt als andere Komponente des Fusionsproteins ein Bereich aus dem C-Terminus des p52 eingesetzt, der etwa 100 bis 150 Aminosäuren des p52-Antigens aufweist. Ganz besonders bevorzugt im Rahmen der vorliegenden Erfindung ist der Bereich aus p52, der die Aminosäuren 297 bis 433 beinhaltet.

Im Rahmen der vorliegenden Erfindung wurde gefunden, daß das Tegumentprotein pp150 mit Seren von gesunden Blutspendern eine sehr hohe IgM-Reaktivität zeigte, wodurch der Wert dieses Antigens für den Nachweis akuter Infek-

EP 0 702 083 A2

tionen deutlich reduziert wird. Ein Anteil von etwa 35 % an positiven Ergebnissen im IgM-Test bei gesunden, seropositiven Blutspendern ist nicht realistisch und zeigt, daß das ganze pp150-Antigen nicht für den Nachweis der IgM-Antikörper geeignet ist.

Weiter wurde gefunden, daß der C-terminale Bereich des p52-Antigens lediglich mit einem Serum eine positive Reaktion zeigt. Würde man allerdings lediglich das p52 als Antigen verwenden, wäre die Sensitivität dieses Antigens nicht ausreichend.

Gegenstand der vorliegenden Erfindung sind daher auch rekombinant hergestellte Fusionsproteine, die wenigstens ein Fragment aus dem C-terminalen Bereich des Tegumentproteins pp150 des Cytomegalievirus (HCMV) und weiterhin wenigstens ein weiteres Fragment aus einem anderen antigenen Protein, das vom humanen Cytomegalievirus stammt, aufweisen.

In einer bevorzugten Ausführungsform stammt das andere antigene Protein des Fusionsproteins von dem als p52 bezeichneten Protein ab, wobei es sich in besonders bevorzugter Ausführungsform um den C-terminalen Bereich des p52-Antigens handelt. Dieser Bereich erstreckt sich in einer ganz besonders bevorzugten Ausführungsform etwa von der Aminosäure an Position 283 bis Aminosäure Nr. 433.

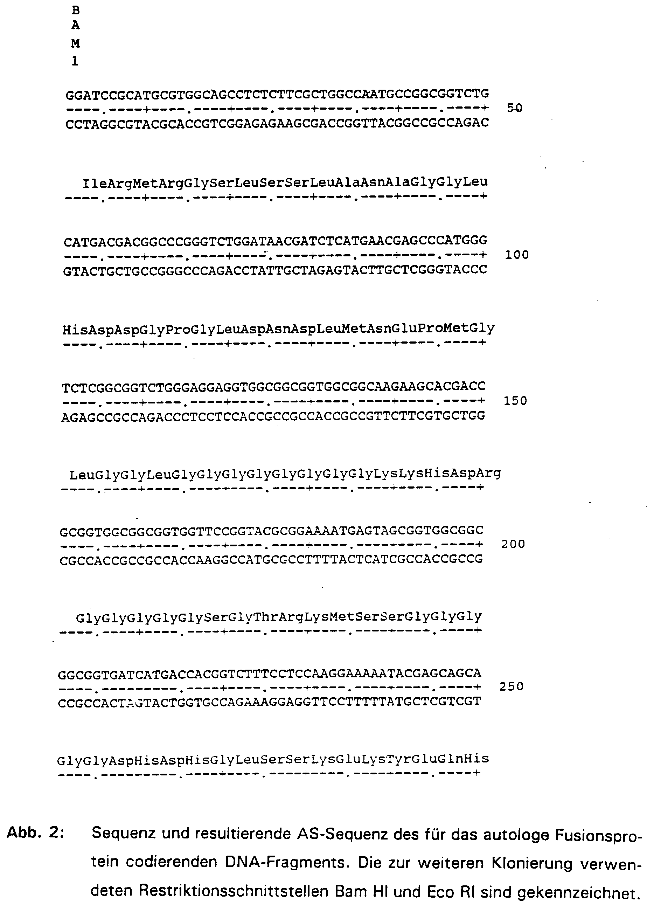

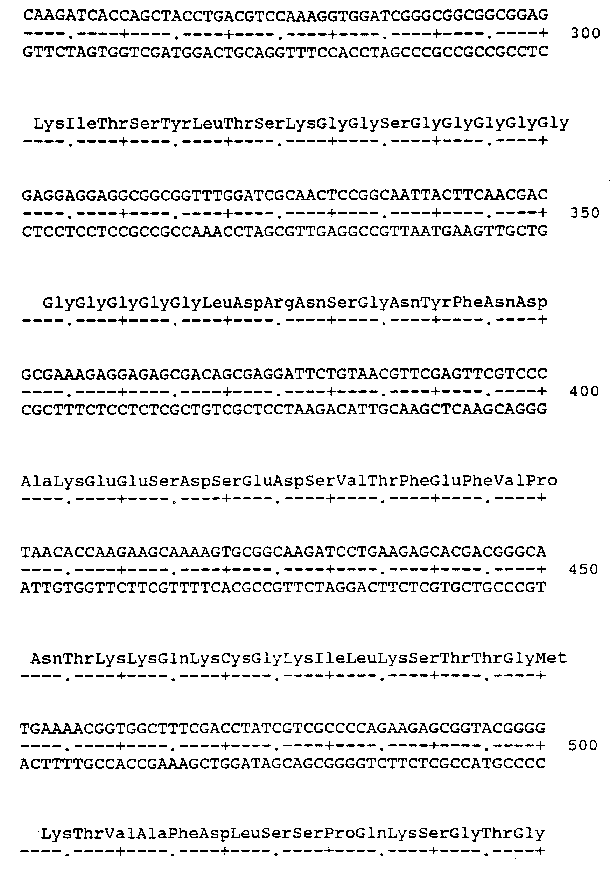

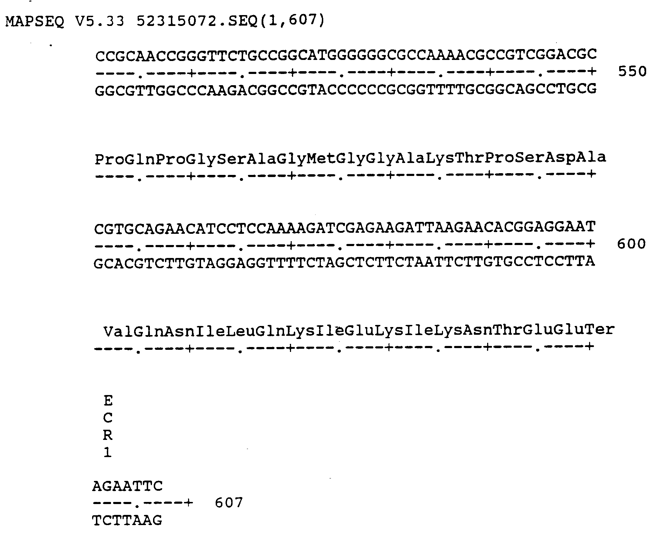

Die Aminosäuresequenz eines besonders bevorzugten Fusionsproteins ist in der Abbildung 2 wiedergegeben. Selbstverständlich können für immunologische Tests Austausche der Aminosäuren durch funktionell gleichwirkende Aminosäuren erfolgen, ohne daß die Wirkung des Fusionsproteins verändert wird. Dem Fachmann ist durchaus bekannt, daß Aminosäureaustausche die immunologischen Eigenschaften eines Proteins nicht berühren, solange die wesentliche Struktur der Epitope nicht verändert wird. Gegenstand der Erfindung sind daher auch solche Fusionsproteine, die eine Homologie von wenigstens 80 % und in besonders bevorzugter Form von wenigstens 90 % zu dem in Abbildung 2 aufgezeigten Fusionsprotein aufweisen. Eine Homologie von 80 % bedeutet, daß 80 von 100 Aminosäuren an den entsprechenden Stellen identisch sind, wohingegen 20 % der Aminosäuren ausgetauscht sein können.

Die eben beschriebenen erfindungsgemäß verwendeten Fusionsproteine bestehen also zum einen aus dem C-terminalen Teil des pp150 und zum anderen aus einem Bereich mit hoher IgM-Reaktivität eines anderen Cytomegalievirus-Antigens. Darüber hinaus können die erfindungsgemäßen Fusionsproteine noch einen kurzen Anteil aufweisen, der durch die rekombinante Herstellung des Fusionsproteins bedingt ist. Dieser Anteil, der insbesondere vom Expressionsvektor stammt, weist aber erfindungsgemäß in bevorzugter Ausführungsform nicht mehr als 25 Aminosäuren auf.

Die so herstellbaren Fusionsproteine weisen eine Aminosäuresequenz eines Polypeptids bzw. eines Fusionsproteins nach einem der Ansprüche 1 bis 15 auf. In bevorzugter Weise stammt ein Teil des Fusionsproteins von der Glutathion-S-Transferase. Die erfindungsgemäßen Fusionsproteine können entweder ein Polypeptid aus dem Leserahmen UL 57 des HCM-Virus aber auch einen Teil von wenigstens einem anderen antigenen Protein des humanen Cytomegalievirus aufweisen. In besonders bevorzugter Ausführungsform handelt es sich bei dem anderen antigenen Protein des humanen Cytomegalievirus um das pp150-Tegumentprotein oder das p52-Protein. Kombinationen von Teilsequenzen der oben erwähnten Proteine sind besonders bevorzugt.

Die vorliegende Erfindung umfaßt auch Testkits zum Nachweis von Antikörpern, insbesondere IgM-Antikörpern gegen das Cytomegalievirus, die dadurch gekennzeichnet sind, daß sie wenigstens ein erfindungsgemäßes Polypeptid und/oder ein erfindungsgemäßes Fusionsprotein umfassen. Hierbei handelt es sich bevorzugterweise um Testkits zur Durchführung eines ELISA-Tests.

In der Diagnostik sind mehrere verschiedene Testmethoden, wie Western Blot, Radio-Immuno-Assay und andere bekannt. Im Rahmen der vorliegenden Erfindung besonders bevorzugt sind solche Testkits, die sich zur Durchführung des ELISA-Tests (Enzyme Linked Immuno Sorbent Assay) eignen. Bei diesen Tests findet eine Kopplung an eine feste Phase statt. Häufig wird das Antigen, im vorliegenden Fall das Fusionsprotein, an eine feste Phase, insbesondere an Mikrotiterplatten gebunden und die unspezifischen Bindungen werden abgesättigt. In diese Mikrotiterplatten wird dann die zu untersuchende Probe, meist Serum, eingebracht, inkubiert und gewaschen. Die an die feste Phase gebundenen Antikörper sind dann gegen das nachzuweisende Antigen gerichtet. Diese Antikörper werden in der Regel mit Anti-Human-Antikörpern nachgewiesen, an die eine Anzeigekomponente gebunden ist. Als besonders geeignet hat sich hierbei die Meerrettich-Peroxidase erwiesen, die eine Farbreaktion katalysiert, anhand der das Testergebnis abgelesen werden kann.

Ein alternatives Verfahren zum Nachweis spezifischer IgM-Antikörper stellt das µ-capture ELISA dar. Dabei wird die feste Phase eines Trägers, z.B. einer ELISA-Platte, mit gegen die µ-Kette des humanen IgM-Moleküls gerichteten polyklonalen bzw. monoklonalen Antikörpern beschichtet. Bei der nachfolgenden Inkubation mit humanem Serum binden IgM-Antikörper selektiv an die feste Phase, während Antikörper aller anderen Antikörperklassen durch Waschen entfernt werden. Die Bestimmung der CMV-spezifischen IgM-Antikörper aus der Vielzahl der gebundenen IgM-Moleküle erfolgt mittels eines enzymmarkierten CMV-Antigens, das durch die gegen dieses Antigen gerichteten IgM-Moleküle gebunden wird. Der gebildete Antikörper-Antigen-Komplex kann - wie oben bereits beschrieben - durch eine enzymvermittelte Farbreaktion nachgewiesen werden.

Gegenstand der vorliegenden Erfindung sind auch Verfahren zum Nachweis von Antikörpern gegen HCMV, bei denen die zu untersuchende Probe mit einem Fusionsprotein zusammengebracht wird. Der Antikörper-Polypeptid- bzw. Fusionsprotein-Komplex kann dann mit einer Nachweiskomponente detektiert werden. Durch die erfindungsgemäßen

4

Polypeptide bzw. Fusionsproteine werden auch Antigene bereitgestellt, die zumindest einen Teil der "zusätzlichen Sensitivität" des pp150 zeigen, gleichzeitig aber eine hohe Spezifität aufweisen. Mit den erfindungsgemäßen Polypeptiden bzw. Fusionsproteinen wird daher nur eine geringe IgM-Reaktivität mit Seren gesunder Blutspender erhalten. Es ist also mit Hilfe der erfindungsgemäßen Polypeptide bzw. Fusionsproteine möglich, einen Test für IgM-Antikörper durchzuführen, der mit einer hinreichenden Sensitivität nachweist, ob eine akute Infektion vorliegt und, der gleichzeitig das Auftreten von falsch positiven Ergebnissen erheblich verringert, wenn nicht gar ausschließt.

Durch die erfindungsgemäßen Fusionsproteine bzw. Peptide werden Antigene bereitgestellt, die eine hohe Sensitivität, gleichzeitig aber eine hohe Spezifität aufweisen. Mit den erfindungsgemäßen Peptiden bzw. Fusionsproteinen wird daher nur eine geringe IgM-Reaktivität mit Seren gesunder Blutspender erhalten. Es ist also mit Hilfe der erfindungsgemäßen Peptide bzw. Fusionsproteine möglich, einen Test für IgM-Antikörper durchzuführen, der mit einer hinreichenden Sensitivität nachweist, ob eine akute Infektion vorliegt und, der gleichzeitig das Auftreten von klinisch nicht relevanten oder gar falsch positiven Ergebnissen erheblich verringert, wenn nicht gar ausschließt.

Bevorzugterweise ist die Nachweiskomponente ein Antihumanantikörper, der gekoppelt ist mit einem Indikatormolekül, insbesondere Meerrettich-Peroxidase.

Die Lehre der vorliegenden Erfindung ermöglicht dem Fachmann auch die Synthese von Oligonucleotiden, die dadurch gekennzeichnet sind, daß sie eine Sequenz von Oligonucleotiden enthalten, die für eine Aminosäuresequenz codieren, die zumindest einem Teil der in Anspruch 1 angegebenen Aminosäuresequenz entspricht. Bevorzugt sind die Oligonucleotide 12-30 Basen lang. Üblicherweise handelt es sich hierbei um DNA-Oligonucleotide, die für die Polymerase Kettenreaktion oder andere die Amplifizierung von Nucleinsäuren ermöglichende Verfahren verwendet werden können.

Gegenstand der vorliegenden Erfindung sind auch Oligonucleotide, die eine Nucleotidsequenz umfassen, die für wenigstens einen Teil des erfindungsgemäßen Polypeptids UL 57/3 codieren. Derartige Oligonucleotide können vorteilhafterweise bei der Polymerase Kettenreaktion als Primer eingesetzt werden. Mit Hilfe der Polymerase Kettenreaktion ist ein empfindlicher Nachweis des Cytomegalievirus möglich. Mit dieser allgemein bekannten Methode gelingt der Nachweis insbesondere in den Fällen, in denen nur eine geringe Kopiezahl der genetischen Information des Virus vorliegt.

Unter dem Begriff "Homologie" wird der Grad der Übereinstimmung von zwei DNA- bzw. Aminosäuresequenzen verstanden. 60 % Homologie bedeutet beispielsweise, daß 60 von 100 Aminosäure-Positionen in den Sequenzen übereinstimmen. Die Homologie von Proteinen wird durch die Sequenzanalyse bestimmt.

Da das humane Cytomegalievirus bereits verhältnismäßig gut erforscht ist, kann die Sequenz sowie der Leserahmen, der das erfindungsgemäße Peptid umfaßt, mit Hilfe der Veröffentlichung von Chee et al., Current Topics in Microbiology and Immunology, **154**, Springer Verlag (1990), S. 126-169, ermittelt werden.

Erfindungsgemäß bevorzugte Fusionsproteine weisen einerseits eine Aminosäuresequenz des erfindungsgemäßen Polypeptids UL 57/3 auf und andererseits ein Fragment eines anderen Proteins, insbesondere eines anderen antigenen Proteins, des humanen Cytomegalievirus und/oder ein vom Expressionsvektor stammendes Fragment. In besonders bevorzugter Weise stammen diese weiteren Bestandteile der Fusionsproteine von dem Tegumentprotein pp150 und/oder von dem Protein p52 des Cytomegalievirus. In ganz besonders bevorzugter Form handelt es sich hierbei um die Fragmente, die in den Beispielen dieser Anmeldung beschrieben werden.

Im Rahmen der vorliegenden Erfindung wurde auch gefunden, daß sich solche Fusionsproteine gut in E.coli exprimieren lassen, die zumindest einen Teil der Glutathion-S-Transferase (GST) aufweisen. Die Vektoren, mit denen derartige Fusionsproteine bereitgestellt werden können, wurden insbesondere von Vornhagen et al., Journal of Clinical Microbiology, **32**, S. 981-986 (1994) beschrieben.

Im Rahmen der vorliegenden Erfindung erfolgte die Evaluierung der Antigene mit:

a) unselektionierten Seren gesunder Blutspender ohne Anzeichen einer akuten HCMV-Infektion. Die Einteilung der Seren in HCMV-positiv/-negativ erfolgte mit einem Paul-Ehrlich-Institut-zugelassenen anti-CMV IgG ELISA (Biotest), siehe Tab. 5

b) ausgesuchten Seren immunkompetenter (funktionierendes Immunsystem) Individuen mit akuter HCMV-Infektion, dokumentiert durch ein positives Ergebnis in der Virusisolierung und/oder durch IgG-Serokonversion (siehe Tab. 3)

c) ausgesuchten Seren von Transplantationspatienten mit akuter HCMV-Primärinfektion, siehe Tab. 4. Der Nachweis der akuten Infektion erfolgte mit Hilfe des pp65-spezifischen Antigenemietests.

Abbildung 1 zeigt die zur Klonierung des autologen Fusionsproteins mit der Bezeichnung 52/3|105/7/2 verwendeten Primer für die Polymerase Kettenreaktion.

Abbildung 2 zeigt die gesamte DNA-Sequenz des in Beispiel 1 beschriebenen Konstruktes und die entsprechende Aminosäuresequenz des hieraus resultierenden Fusionsproteins.

Abbildung 3 zeigt die Fotographie eines SDS-Polyacrylamidgeles, das die Expression und Reinigung des rekombinanten Fusionsproteines 52/3|150/7/2 belegt.

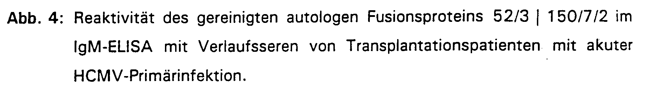

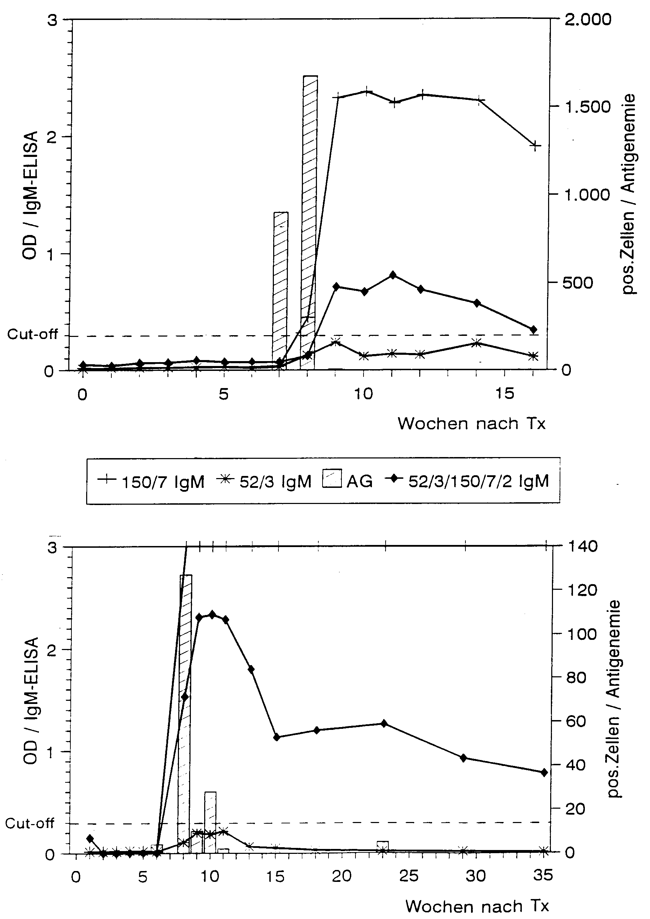

Abbildung 4 zeigt die IgM-Verläufe von zwei Patienten (obere bzw. untere Abbildung), bei denen im IgM-ELISA einmal ein C-terminales Fragment von pp150 (Aminosäure 862-1048) und einmal das C-terminale Fragment von p52 (Aminosäure 297-433) eingesetzt wurde. Weiterhin wurde das erfindungsgemäße Fusionsprotein 52/3|150/7/2 eingesetzt. Die mit AG bezeichneten schraffierten Säulen stellen die mit anderen Methoden festgestellten Antigenemiewerte dar. Zu diesen Zeitpunkten konnte eine akute Infektion beobachtet werden. Abbildung 4 zeigt deutlich, daß mit dem erfindungsgemäßen Fusionsprotein ein eindeutiger IgM-Nachweis möglich ist, was beispielsweise mit dem Fragment 52/3 allein nicht möglich ist.

Abbildung 5 zeigt die Tabelle 1, bei der die IgM-Reaktivität verschiedener rekombinanter Antigene verglichen wird einmal mit den Seren seronegativer, gesunder Blutspender und andererseits mit den Seren seropositiver, gesunder Blutspender. Der von dem Tegumentprotein pp150 stammende Fragmentanteil (Aminosäure 862-1048 $\cong$ 150/7) weist eine hohe, offensichtlich nicht krankheitskorrelierte IgM-Reaktivität auf. Bei Verwendung des erfindungsgemäßen Fusionsproteins (52/3|105/7/2) wurde die Anzahl der IgM-positiven Seren erheblich reduziert.

Abbildung 6 zeigt in Tabelle 2 die OD-Werte im IgM-ELISA mit Seren verschiedener immunkompetenter Patienten mit akuter HCMV-Infektion. Im Vergleich zum rekombinanten Antigen p52/3 zeigt das erfindungsgemäße Fusionsprotein eine deutlich verbesserte Sensitivität.

Abbildung 7 zeigt die zur Klonierung der verschiedenen Fragmente verwendeten Primer bzw. Oligonucleotide für die Polymerase Kettenreaktion (PCR).

Abbildung 8 zeigt die DNA-Sequenz und die daraus resultierende Aminosäuresequenz des erfindungsgemäßen Peptids aus dem Leserahmen UL 57, das die Bezeichnung UL 57/3 erhalten hat.

Abbildung 9 zeigt die DNA und die daraus resultierende Aminosäuresequenz des autologen Fusionsproteins mit der Kurzbezeichnung 52/3 57/3. Der andere Teil des Fusionsproteins stammt von dem antigenen Protein p52 des humanen Cytomegalievirus.

Abbildung 10 zeigt die DNA und die daraus resultierende Aminosäuresequenz des autologen Fusionsproteins 52/3 57/3 150/7/2. Dieses Fusionsprotein enthält neben der Sequenz des erfindungsgemäßen Peptids jeweils einen Teil des antigenen Proteins p52 und des Tegumentproteins pp150.

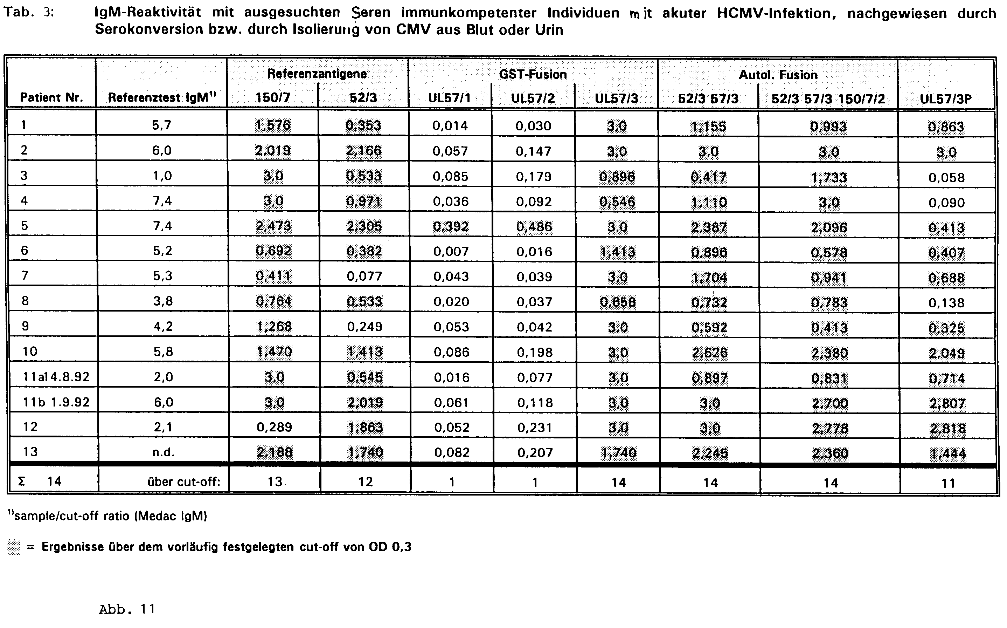

Abbildung 11 stellt in Tabelle 3 die IgM-Reaktivität mit verschiedenen Seren immunkompetenter Individuen mit akuter HCMV-Infektion dar. Bei den Patientenseren handelt es sich um solche Patienten, die über ein funktiolierendes Immunsystem verfügen und eine akute HCMV-Infektion haben.

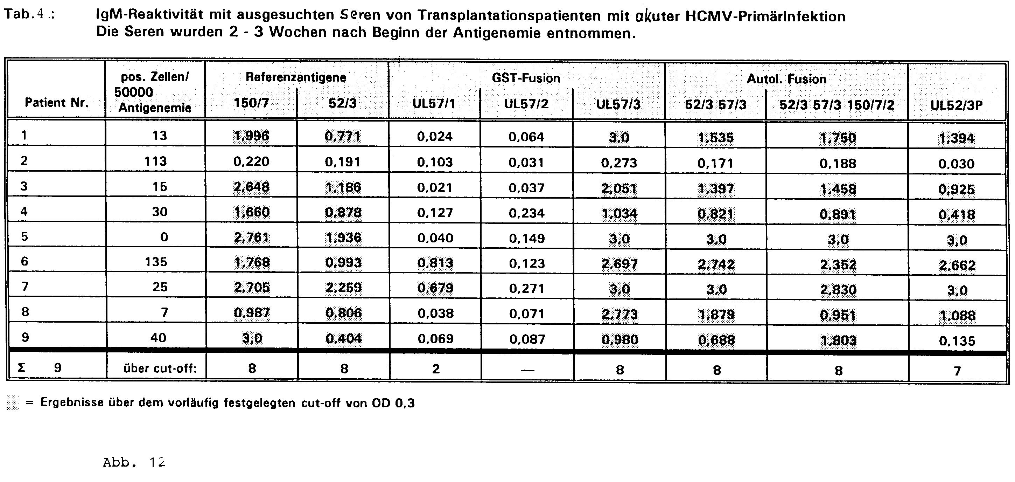

Abbildung 12 zeigt in Tabelle 4 die IgM-Reaktivität mit Seren von Transplantationspatienten mit akuter HCMV-Primärinfektion. Transplantationspatienten verfügen regelmäßig nicht über ein voll funktionierendes Immunsystem, da in Zusammenhang mit der Transplantation regelmäßig Immunsuppressiva verabreicht werden.

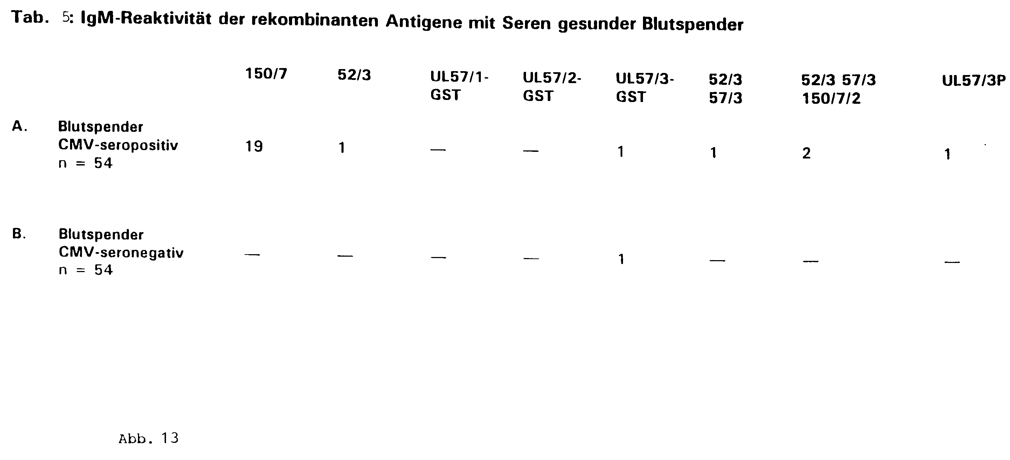

Abbildung 13 zeigt in Tabelle 5 die IgM-Reaktivität der rekombinanten Antigene mit den Seren gesunder Blutspender, die unterteilt wurden einmal in CMV-seropositive und andererseits in CMV-seronegative. Die Unterscheidung in Seropositivität bzw. Seronegativität erfolgte durch die Bestimmung der IgG-Antikörper. Vorteilhaft bei den erfindungsgemäßen Peptiden bzw. Fusionsproteinen ist, daß praktisch keine falsch positiven Ergebnisse erhalten wurden.

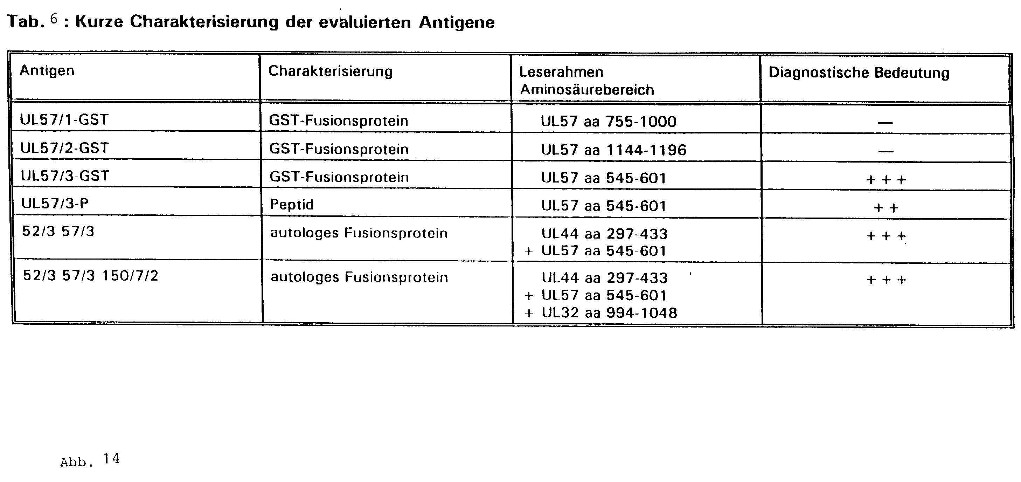

Abbildung 14 zeigt in Tabelle 6 eine kurze Charakterisierung der erfindungsgemäßen Antigene. Die Abkürzung GST bedeutet hierbei, daß der andere Bestandteil des Fusionsproteins von der Glutathion-S-Transferase herstammt. Die Abkürzung P bedeutet, daß es sich um ein chemisch synthetisiertes Peptid handelt.

Die Daten zeigen deutlich, daß von den getesteten UL57-Fragmenten insbesondere UL57/3 diagnostisch relevant ist. Dieses Antigenfragment zeigte mit Seren von Personen mit akuter HCMV-Infektion eine extrem hohe IgM-spezifische Reaktivität. Die Zahl der reaktiven Proben war bei den Immunkompetenten (Tab. 3) wie auch bei den Transplantationspatienten (Tab. 4) gleich bzw. höher als mit den rekombinanten Antigenen 150/7 und 52/3. Diese Antigene hatten sich in einer vorangegangenen Evaluierung als besonders geeignet für den Nachweis akuter HCMV-Infektionen erwiesen. Mit Seren gesunder Blutspender zeigte sich hingegen eine sehr geringe IgM-Reaktivität. Bei der Austestung der autologen Fusionsproteine ergaben sich bei beiden Kollektiven der akut-Infizierten eine gleiche Anzahl an positiven Ergebnissen. Lediglich bei der Höhe der Reaktivität konnten individuelle Unterschiede festgestellt werden. Eine etwas geringere IgM-spezifische Prevalenz war beim chemisch synthetisierten Peptid 57/3-P festzustellen. Zusammenfassend ist festzustellen, daß das mit UL57/3 bezeichnete Antigenfragment hervorragend zur IgM-spezifischen Serodiagnostik akuter HCMV-Infektionen geeignet ist.

**Beispiel 1**

**Klonierung des autologen Fusionsproteins 52/3|150/7/2**

Es handelt sich hier um ein autologes Fusionsprotein, das den kompletten Bereich von 52/3 und die 54 C-terminalen Aminosäuren von 150/7 kombiniert.

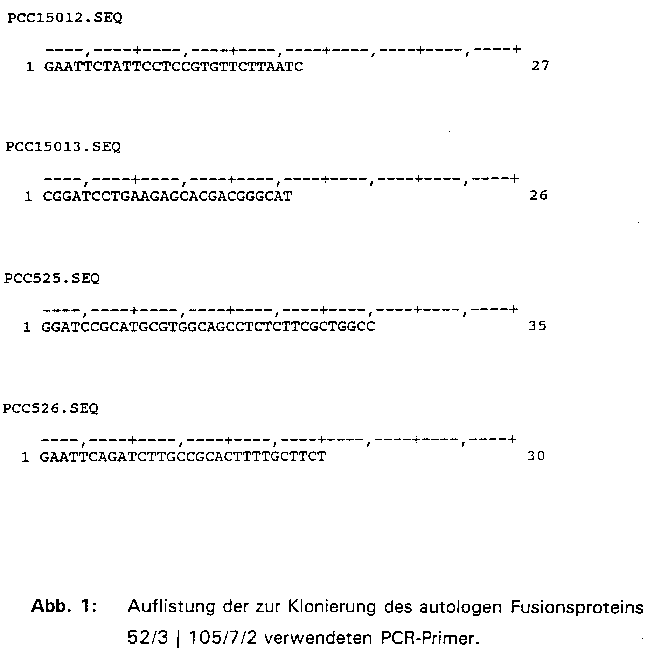

**1.** Ausgehend vom Klon puc8/PCC150/7, der die Teilsequenz von pp150, der für die AS 862-1048 kodiert, enthält, erfolgte eine PCR-Amplifikation des Teilfragmentes PCC150/7/2, mit Hilfe der Primer PCC15012.seq und PCC15013.seq (Abb. 1). Beide Primer besitzen zusätzlich zum zu pp150 komplementären Bereich Überhänge, die bestimmte singuläre Restriktionsschnittstellen enthalten, d.h. PCC15012.seq: EcoRI, PCC15013.seq: BamHI. Diese Schnittstellen ermöglichen die gerichtete Klonierung des Amplifikats.

Die PCR-Amplifikation erfolgte in einem Gesamtvolumen von 100 µl bestehend aus 10 µl Reaktionspuffer (Perkin-Elmer Cetus), 200 µM der 4 Desoxynucleotide, 0,5 µM beider PCR-Primer, 50-100 ng der Ausgangs-DNA sowie 2,5 Units der AmpliTaq DNA-Polymerase (Perkin-Elmer Cetus). Die Amplifikation wurde im Perkin-Elmer Cetus DNA Thermal Cycler in 25 Zyklen bei folgenden Bedingungen durchgeführt: 1 min - 55°C, 1 min - 72°C, 1 min - 94°C. Der PCR-Reaktionsansatz wurde in einer Submarine-Agarose Gelkammer in einem 1,2 %igen Agarosegel (Ultra Pure Agarose, BRL), das 0,5 µg/ml Ethidiumbromid enthält unter Verwendung eines TBE-Laufpuffers (0,089 M Tris/Borat, 0,002 M EDTA) elektrophoretisch aufgetrennt. Danach wurde das amplifizierte DNA-Fragment mit einer UV-Leuchte bei 364 nm sichtbar gemacht und der Gelbereich, der die entsprechende Bande enthielt, mit einem Skalpell herausgeschnitten. Die Elution der DNA aus dem Gelfragment erfolgte mittels einer Biotrap Kammer (Schleicher & Schüll) entsprechend der Vorschrift des Herstellers. Daran schloß sich eine zusätzliche Aufreinigung der DNA mit einer Elutip D-Säule (Schleicher & Schüll) entsprechend der Gebrauchsanweisung des Herstellers an.

Das so gereinigte und getrocknete DNA-Fragment wurde in 80 µl aqua dest gelöst. Nach Zugabe von 10 µl NEBuffer 4 (New England Biolabs) erfolgte ein BamHI/EcoRI Verdau durch Zugabe von jeweils 100 U beider Enzyme. Nach 2 Stunden folgte eine Phenol-Extraktion mit anschließender Ethanolprezipitation. 100 ng des so vorbereiteten DNA-Fragments wurden mit 200 ng BamHI/EcoRI-behandelter DNA des Standardvektors puc8 für 16h bei 4°C ligiert und anschließend wurde damit E.coli JM109 transformiert. Die Ausplattierung des Transformationsansatzes erfolgte auf mit Ampicillin (50 µg/ml) und X-Gal (30 µg/ml) versetzten Agarplatten. Weiße Kolonien wurden in 3 ml LB-Medium mit Ampicillin überimpft und für 12-16h bei 37°C unter Schütteln inkubiert. Nach Isolierung der Plasmid-DNA erfolgte ein EcoRI/BamHI Restriktionsverdau und eine Elektrophorese im Agarosegel. Ein Klon, bei dem sich ein zusätzliches DNA-Fragment der erwarteten Größe zeigte, erhielt den Namen puc8/PCC150/7/2 und wurde zur weiteren Klonierung verwendet.

**2.** Ausgehend vom Klon puc8/PCC52/3 - das klonierte Fragment kodiert für AS 297-433 von p52 - erfolgte PCR-Amplifikation mit Hilfe der Primer PCC525.seq und PCC526.seq und die spätere Klonierung des amplifizierten Fragments wie oben beschrieben. Aufgrund der Überhänge der beiden hier verwendeten Primer besitzt das amplifizierte Fragment am 5'-Ende eine BamHI und am 3'-Ende jeweils eine BglII- und EcoRI-Schnittstelle. Der entsprechende Klon wurde als puc8/PCC52/3F bezeichnet.

**3.** Mit der DNA des Klons puc8/PCC150/7/2 erfolgte ein Restriktionsverdau mit BamHI und EcoRI. Das dabei freigesetzte Fragment von ca. 160 bp wurde mit Hilfe einer Agarose-Elektrophorese isoliert und wie oben beschrieben aus dem herausgeschnittenen Gelstück eluiert. Ca. 50 ng dieses DNA-Fragmentes wurden dann mit 200 ng des mit BglII und EcoRI geöffneten und ebenfalls mittels Elektrophorese gereinigten Vektors puc8/PCC52/3F ligiert. Daran schloß sich eine Transformation mit E.coli JM109 und eine Ausplattierung auf Ampicillin enthaltenden Agarplatten an. Isolierte Kolonien wurden in 3 ml LB-Medium mit Ampicillin überimpft und 12-16h unter Schütteln bei 37°C inkubiert. Nach Isolierung der Plasmid-DNA erfolgte ein BamHI/EcoRI-Restriktionsverdau mit anschließender Elektrophorese im Agarosegel. Ein Klon mit einem Insert der erwarteten Größe wurde als puc8/52/3 150/7/2 bezeichnet und für die weiteren Experimente verwendet. Bei dieser Vorgehensweise wird ausgenutzt, daß die Erkennungssequenzen von BamHI und BglII die gleichen Überhänge besitzen, wodurch eine Ligation ermöglicht wird. Beide Schnittstellen gehen jedoch nach erfolgter Ligation verloren. Der Übergang von 52/3F zu 150/7/2 ist so gewählt, daß eine Translation im gleichen Leserahmen möglich ist. Abb. 2 zeigt die gesamte DNA-Sequenz des oben beschriebenen Konstrukts und die entsprechende AS-Sequenz des resultierenden autologen Fusionsproteins.

**4.** Um die Expression des autologen Fusionsproteins zu gewährleisten, erfolgte die Umklonierung des entsprechenden DNA-Fragmentes in den Vektor pET5c, der den starken T7-Promotor des Gens 10 des Bakteriophagen T7 besitzt. Dahinter besitzt dieser Vektor eine Ribosomenbindungsstelle und ein Startcodon in entsprechendem Abstand. Hinter dem Startcodon liegt ein Leserahmen von 11 Aminosäuren des Gens 10 des Bakteriophagen T7 und eine BamHI- und eine EcoRI- Schnittstelle. Das Leseraster der BamHI-Schnittstelle stimmt mit der des oben beschriebenen autologen Fusionsproteins überein. 100 ng des mittels EcoRI- und BamHI-Restriktionsverdau aus puc8/52/3|150/7/2 freigesetzten DNA-Fragments wurden mit 200 ng des mit den gleichen Restriktionsenzymen geöffneten Vektors für 16h bei 4°C ligiert. Danach folgte eine Transformation mit E.coli JM109 und eine Ausplattierung auf Ampicillin-haltigen Platten. Isolierte Kolonien wurden in 3 ml LB-Medium mit Ampicillin überimpft und 16h unter Schütteln bei 37°C inkubiert. Nach Isolierung der Plasmid-DNA erfolgte ein BamHI/EcoRI-Restriktionsverdau. Ein Klon mit einem Insert der zu erwartenden Größe wurde als pET5c/52/3|150/7/2 bezeichnet. Um die Expression des rekombinanten Proteins zu gewährleisten, wurde DNA des Expressionsvektors in den chloramphenicolresistenten Expressionsstamm BL21(DE3)pLysS transformiert. Einer der resultierenden Klone wurde für die weiteren Arbeiten verwendet.

**Beispiel 2**

**Expression von rekombinantem Fusionsprotein 52/3|150/7/2**

**1.** Ausgehend von einer Plattenkolonie des Klons pET5c/52/3|150/7/2 in BL21(DE3)pLysS wurde eine 15 ml Flüssigkultur - LB-Medium mit Ampicillin (Amp) und Chloramphenicol (CA) - bei 37°C im Rundschüttler bis zu einer optischen Dichte (600 nm) von 1.8-2.0 angezogen. Die Kultur wurde dann mit Glycerin (87 %) bis zu einer Endkonz. von 15 % (v/v) versetzt, in 0.1 ml Portionen aliquotiert und bei -60 bis -80°C bis zur weiteren Verwendung gelagert.
**2.** Ein gefrorenes Aliquot der Glycerinkultur wurde rasch aufgetaut und in 150 ml LB/CA,AMP-Medium einpipettiert. Die Kultivierung dieser Über-Nacht-Kultur erfolgte in einem 1 l Erlenmeyerkolben (EMK) im Rundschüttler bei 28°C und 100 Upm für 16h.
**3.** Die Anzucht der 6 l Hauptkultur erfolgte in 12 Parallelansätzen zu je 0.5 l in 2 l EMK mit Schikanen. Das Medium (LB/CA,AMP) wurde auf 37°C vortemperiert. Nach Innokulation der Kolben mit je 10 ml der Vorkultur (1:51) erfolgte die Inkubation bei 37°C und 160 Upm im Rundschüttler. Das Wachstum wurde kontinuierlich durch Messung der OD bei 600 nm verfolgt. Bei einer OD von 0.6 wurde die Expression des rekombinanten Antigens durch Zugabe von IPTG bis zu einer Endkonzentration von 1 mM induziert.
**4.** Die Ernte erfolgte 3h nach Induktion durch Zentrifugation (6x1 l-Becher, 4000 g, 0-4°C, 30 min). Die gut abgetropften Bakterienpellets wurden in 200 ml eiskaltem PBS resuspendiert und erneut zentrifugiert (2x250 ml-Becher, 5000 g, 0-4°C, 10 min). Die erneut gut abgetropften Pellets wurden eingefroren und bei -20 bis -30°C bis zur weiteren Verarbeitung gelagert.
**5.** Vor der Induktion und vor der Ernte der Bakterien wurden 1.5 ml Aliquots der Bakteriensuspension aus ausgewählten Kolben entnommen, in ein Eppendorf-Gefäß überführt und die Bakterien durch Zentrifugation pelletiert. Die Bakterien wurden anschließend mit SDS-Elektrophoresepuffer behandelt und ein Aliquot einer Analyse in der SDS-Elektrophorese unter Verwendung eines 17.5 %igen Polyacrylamidgels unterzogen. Die vor der Ernte gezogenen Proben zeigten im Vergleich zu den vor der Induktion gezogenen Proben eine zusätzliche, stark ausgeprägte Proteindoppelbande im Bereich von 25k.

**Beispiel 3**

**Aufschluß der Bakterien**

Die eingefrorenen Bakterienpellets wurden aufgetaut, in 160 ml Basis-Puffer (Tris-HCl/20mM/pH7.5) resuspendiert und anschließend mit einem Teflon/Glas-Potter-Homogenisator homogenisiert. Unter Rühren wurden danach folgende Additive zugegeben: NP-40 (0.05 %), PMSF (0.2 mM), Pefabloc (0.2 mM), EDTA (50 mM) und Lysozym (50 mg) bis zu einem Gesamtvolumen von 200 ml. Der Ansatz wurde unter starkem Rühren für 60 min bei Raumtemperatur inkubiert und anschließend sofort auf Eis gestellt. Alle weiteren Schritte erfolgten auf Eis bzw. gekühlt. Nach der Inkubation wurde Glycerin (10 %) und 2-Mercaptoethanol (14 mM) zugegeben und das Volumen mit Basis-Puffer auf 280 ml eingestellt. Der Lyseansatz wurde anschließend sonifiziert (20 kHz, pulsierend, 5 min, Titanrüssel 3/4"). Nicht solubilisiertes Material wurde durch Zentrifugation entfernt (2x250 ml-Becher, 27000 g, 30 min, 0-4°C). Die Pellets wurden verworfen.

**Beispiel 4**

**Reinigung des rekombinanten Proteins 52/3|150/7/2**

**1.** Dem Überstand aus Beispiel 3 wurde unter Rühren im Eisbad festes, fein gemörsertes Ammoniumsulfat bis zu einer Konzentration von 25 % Sättigung langsam zugegeben. Es schloß sich eine Inkubation für 15 min unter Rühren an. Nach Zentrifugation (2x250 ml-Becher, 27000 g, 30 min, 0-4°C) wurden die Pellets verworfen. Der Überstand wurde erneut mit Ammoniumsulfat bis zur Konzentration von 45 % Sättigung versetzt und wie vorher zentrifugiert. Der Überstand wurde verworfen. Die Pellets wurden in 25 ml Basis-Puffer, der zusätzlich 2-Mercaptoethanol (14 mM) und Pefabloc (0.1 mM) enthält, resuspendiert, eingefroren und über Nacht gelagert bei -20 bis -30°C.
**2.** Die mit Ammoniumsulfat (25-45 %) fraktionierte Protein-Lösung wurde aufgetaut und präzipitiertes Protein durch Zentrifugation (1x50 ml-Becher, 40000 g, 30 min, 0-4°C) entfernt. Der Überstand wurde über eine Sephadex G-25 (coarse) Säule (Volumen mindestens 200 ml) chromatographiert, wobei A (280 nm) und die Leitfähigkeit im Durchfluß gemessen werden. Als Säulenpuffer wurde Basispuffer mit 2-Mercaptoethanol (1.4 mM) und Pefabloc (0.02 mM) verwendet. Das Protein im Ausschlußvolumen wurde vollständig gesammelt und sofort über SP-Sepharose (Fast Flow) chromatographiert. Dabei wurde eine Säule der Dimension 2.6 x 12 cm (60 ml) verwendet. A (280 nm) und Leitfähigkeit wurden kontinuierlich registriert. Der Fluß betrug 5 ml/min, als Säulenpuffer wurde Basispuffer mit 2-Mercaptoethanol (1.4 mM) und Pefabloc (0.02 mM) verwendet. Nach Probenauftrag, gefolgt von 100 ml Säulenpuffer, wurde ein linearer NaCl-Gradient (dC/dV = 1 mM/ml, bis 300 mM) in Säulenpuffer angelegt. Das Eluat wurde in 10 ml-Fraktionen gesammelt und eingefroren.

Die Antigen-enthaltenden Fraktionen befanden sich im Bereich zwischen 100-200 mM NaCl. Der Nachweis erfolgte durch SDS-PA-Disc-Elektrophorese mit anschließender Coomassie-Färbung.

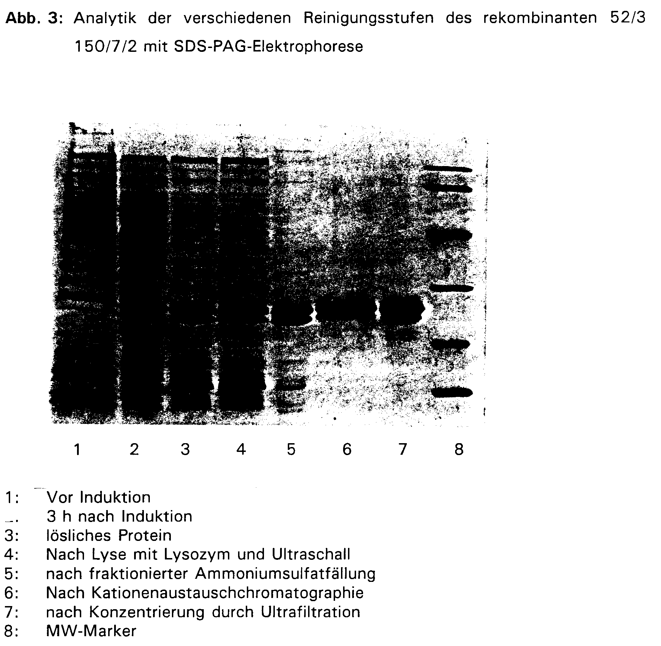

3. Die Fraktionen, die nachweislich reines Antigen enthielten (Coomassie-Gel), wurden aufgetaut, vereint, mit Glycerin versetzt (20 %) und mittels Ultrafiltration (Rührzelle, Eisbad, Membran = Omega 30) konzentriert bis zu einer Endkonzentration von mindestens 2 mg/ml. Das Retentat wurde der Kammer entnommen und aliquotiert eingefroren und gelagert bei -60 bis -80°C. Der Verlauf der Reinigung ist in Abb. 3 dokumentiert.

## Beispiel 5

### Reaktivität des gereinigten rekombinanten Antigens 52/3|150/7/2 im ELISA

**1.** Testdurchführung

Je 100 ng des gereinigten rekombinanten Antigens gelöst in 100 µl 0,01 M Carbonatpuffer pH 9,5 wurden in die Vertiefungen von Mikrotiterplatten (Nunc) gegeben und 16h in einer geerdeten feuchten Kammer inkubiert. Nach Zugabe von 100 µl einer Kalbserum enthaltenden Nachbeschichtungslösung wurde die Inkubation für 2 weitere Stunden fortgesetzt. Danach wurden die Platten entleert und sorgfältig ausgeschlagen. Anschließend wurden die Platten für die eigentliche Testdurchführung verwendet oder nach Trocknung im Vakuumschrank und anschließendem Einschweißen in Folienschlauch bis zum späteren Gebrauch bei -20°C gelagert. Zur eigentlichen Testdurchführung wurden die Testnäpfchen der ELISA-Platten mit 100 µl 1:21 verdünntem Serum gefüllt, mit einer Plastikfolie abgeklebt und 1h bei 40°C schwimmend im Wasserbad inkubiert. Nach dreimaligem Waschen im Biotest ELISA Washer II erfolgte die Inkubation mit 100 µl eines gegen humanes IgM gerichteten, Peroxidase-markierten, monoklonalen Antikörper aus der Maus (Janssen) für 30 min bei 40°C. Nach abermaligem Waschen wurden die gebundenen Antikörper durch eine Farbreaktion mit 1,2-Phenyldiamin als Chromogen sichtbar gemacht. Die optische Dichte der einzelnen Proben wurde bei 495 nm (Referenz: 620 nm) am Anthos HTII ELISA-Reader ermittelt. Alle OD-Werte größer als 0,3 wurden als positives Ergebnis gewertet.

**2.** Ergebnisse

Die Überprüfung der Reaktivität erfolgte mit:

- unselektionierten Seren gesunder Blutspender ohne Anzeichen einer akuten HCMV-Infektion. Die Einteilung dieser Seren in HCMV-positiv/negativ erfolgte mit einem zugelassenen anti-CMV IgG ELISA (Biotest)
- ausgesuchten Seren immunkompetenter Individuen mit akuter HCMV-Infektion
- ausgesuchten Verläufen von Nierentransplantationspatienten mit akuter HCMV-Infektion.

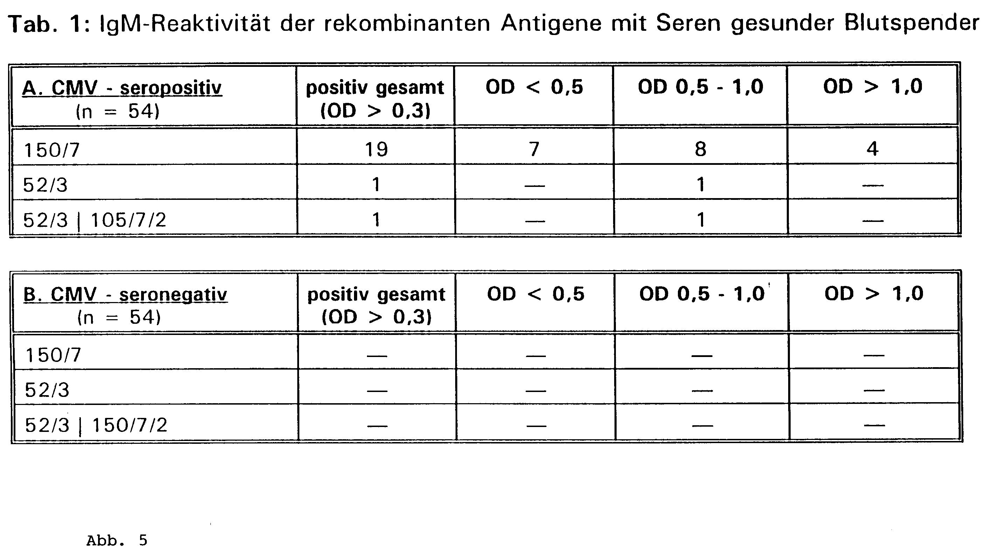

Tab. 1 in Abb. 5 zeigt die IgM-Reaktivität des autologen Fusionsproteins 52/3|150/7/2 mit Seren HCMV-seropositiver (n=54) bzw. seronegativer, gesunder Blutspender im Vergleich zu den rekombinanten Antigenen 52/3 und 150/7, die ebenfalls in pET5c exprimiert und bis zur Homogenität gereinigt wurden. Während 150/7 eine hohe, offensichtlich nicht krankheitskorrelierte IgM-Reaktivität (> 32 %) mit HCMV-positiven Seren zeigte, wies das autologe Fusionsprotein die gleiche geringe Reaktivität wie 52/3 und somit eine deutlich bessere Spezifität als 150/7 auf.

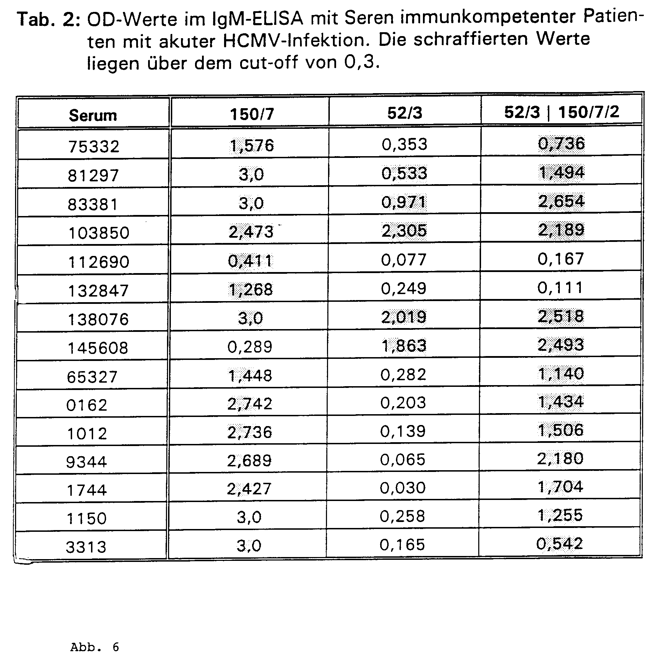

Tab. 2 in Abb. 6 zeigt die OD-Werte im oben beschriebenen IgM-ELISA mit 15 Seren immunkompetenter Personen mit akuter HCMV-Infektion. Alle Seren waren im konventionellen IgM-ELISA positiv. Das autologe Fusionsprotein 52/3|150/7/2 zeigte im Vergleich zum rekombinanten Antigen 52/3 eine deutlich verbesserte Sensitivität, d.h. zahlreiche der mit 52/3 negativen Seren ergaben mit dem autologen Fusionsprotein OD-Werte über dem Cut-off-Wert von 0,3.

Abb. 4 zeigt die Ergebnisse der rekombinanten Proteine 52/3|150/7/2, 52/3 und 150/7 im oben beschriebenen IgM-ELISA mit Serumverläufen von zwei Transplantationspatienten mit akuter HCMV-Primärinfektion. Beide Patienten waren HCMV-seronegativ vor der Transplantation und erhielten ein Organ (Niere) eines seropositiven Spenders. Der Verlauf der akuten Infektion wurde mit dem pp65-spezifischen Antigenemietest und der PCR verfolgt. In beiden Verläufen zeigte 52/3 keine bzw. nur eine schwache IgM-Reaktivität, während 150/7 eine starke Reaktivität zeigte. Bei beiden Patienten ergab sich beim autologen Fusionsprotein 52/3|150/7/2 eine hohe IgM-spezifische Seroreaktivität, die mit dem akuten Krankheitsverlauf korrelierte.

**Beispiel 6**

PCR-Amplifikation und Klonierung des Fragmentes UL57/3

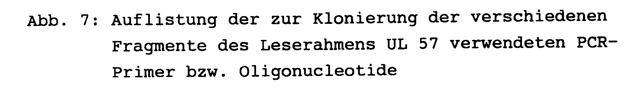

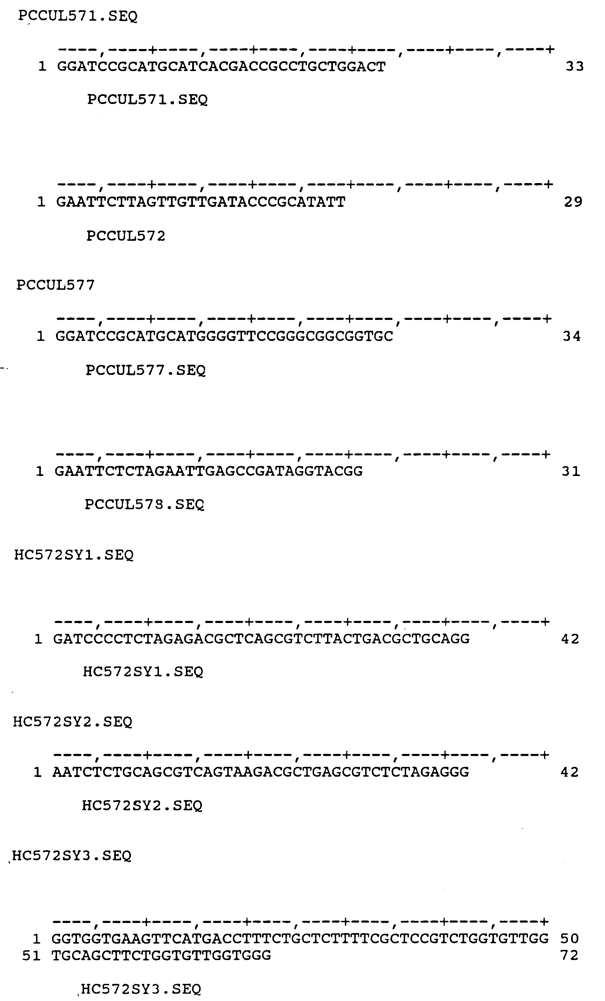

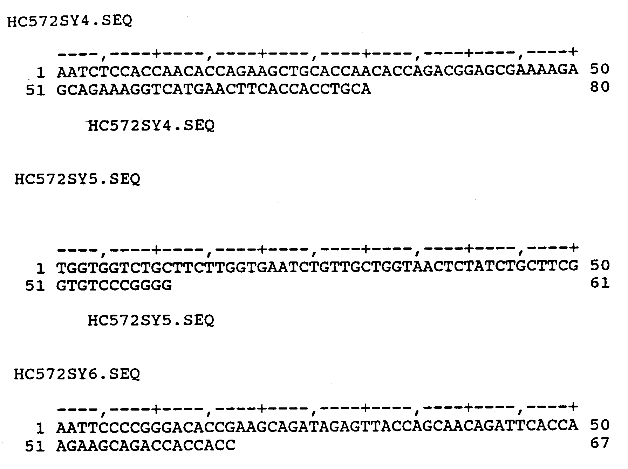

Ausgehend vom Cosmid-Klon PCM1029 (Fleckenstein, B., I. Mueller and J. Collins. 1982, Cloning of the complete human cytomegalovirus in cosmids. Gene 18, 39-46), der die Gesamtsequenz von UL57 enthält, erfolgte eine PCR-Amplifikation des Teilfragmentes UL57/3, das für die Aminosäuren 545-601 des Leserahmens UL57 codiert, mit Hilfe der Primer PCCUL577.seq und PCCUL578.seq (Abb. 7). Beide Primer besitzen zusätzlich zum UL57 komplementären Bereich Überhänge, die bestimmte singuläre Restriktionsschittstellen enthalten, d.h PCCUL578.seq: EcoRI, PCCUL577.seq: BamHI. Diese Schnittstellen ermöglichen die gerichtete Klonierung des Amplifikats. Die PCR-Amplifikation erfolgte in einem Gesamtvolumen von 100 µl, bestehend aus 10 µl Reaktionspuffer (Perkin-Elmer Cetus), 200 µM der 4 Desoxynucleotide, 0,5 µM beider PCR-Primer, 50-100 ng der Ausgangs-DNA sowie 2,5 Units der AmpliTaq DNA-Polymerase (Perkin-Elmer Cetus). Die Amplifikation wurde im Perkin-Elmer Cetus DNA Thermal Cycler in 25 Zyklen bei folgenden Bedingungen durchgeführt: 1 min -55°C, 1 min -72°C, 1 min 94°C. Der PCR-Reaktionsansatz wurde in einer Submarine-Agarose Gelkammer in einem 1,2 %igen Agarosegel (Ultra Pure Agarose, BRL), das 0,5 µg/ml Ethidiumbromid enthält, unter Verwendung eines TBE-Laufpuffers (0,089M Tris/Borat, 0,002M EDTA) elektrophoretisch aufgetrennt. Danach wurde das amplifizierte DNA-Fragment mit einer UV-Leuchte bei 364 nm sichtbar gemacht und der Gelbereich, der die entsprechende Bande enthielt, mit einem Skalpell herausgeschnitten. Die Elution der DNA aus dem Gelfragment erfolgte mittels einer Biotrap Kammer (Schleicher & Schüll), entsprechend der Vorschrift des Herstellers. Daran schloß sich eine zusätzliche Aufreinigung der DNA mit einer Elutip D-Säule (Schleicher & Schüll), entsprechend der Gebrauchsanweisung des Herstellers, an.

Das so gereinigte und getrocknete DNA-Fragment wurde in 80 µl aqua dest gelöst. Nach Zugabe von 10 µl NEBuffer 4 (New England Biolabs) erfolgte ein BamHI/EcoRI- Verdau durch Zugabe von jeweils 100 U beider Enzyme. Nach 2 Stunden folgte eine Phenol-Extraktion, mit anschließender Ethanolprezipitation. 100 ng des so vorbereiteten DNA-Fragments wurden mit 200 ng BamHI/EcoRI-behandelter DNA des Standardvektors pUC8 für 16h bei 4°C ligiert und anschließend damit E.coli JM109 transformiert. Die Ausplattierung des Transformationsansatzes erfolgte auf mit Ampicillin (50 µg/ml) und X-Gal(30 µg/ml) versetzten Agarplatten. Weiße Kolonien wurden in 3 ml LB-Medium mit Ampicillin überimpft und für 12-16 h bei 37°C unter Schütteln inkubiert. Nach Isolierung der Plasmid-DNA erfolgte ein EcoRI/BamHI Restriktionsverdau und eine Elektrophorese im Agarosegel. Ein Klon, bei dem sich ein zusätzliches DNA-Fragment der erwarteten Größe zeigte, erhielt den Namen pUC8/PCCUL57/3 und wurde zur weiteren Klonierung verwendet.

**Beispiel 7**

PCR-Amplifikation und Klonierung des Teilfragmentes UL57/1

Ausgehend vom Klon PCM1029 (s. Beispiel 6) erfolgte die PCR-Amplifikation des Fragmentes UL57/1, das für die Aminosäuren 755-1000 des Leserahmens UL57 codiert, mit Hilfe der Primer PCCUL571.seq und PCCUL572.seq und die spätere Klonierung des amplifizierten Fragments wie oben beschrieben. Der entsprechende Klon wurde als puc8/PCCUL571 bezeichnet.

**Beispiel 8**

Klonierung des Teilfragmentes UL57/2

Bei diesem Teilfragment war eine PCR-Amplifikation trotz mehrfacher Modifikation der Primer nicht möglich gewesen. Aus diesem Grunde wurde die zu klonierende Ausgangs-DNA chemisch synthetisiert. Dazu wurde die Aminosäuresequenz des Fragments mittels des Computerprogramms der Fa. DNASTAR in DNA übersetzt, wobei lediglich Codons stark exprimierter E.coli-Gene verwendet wurden. Durch geringfügige Modifikation der DNA-Sequenz wurden, ohne die AS-Sequenz zu verändern, singuläre Restriktionsschnittstellen eingeführt, um die Klonierung der chemisch synthetisierten Oligonukleotidpaare zu ermöglichen.

Die Synthese der Oligonukleotide erfolgte auf einem DNA-Synthesizer 381A der Fa. Applied Biosystems, die anschließende Aufreinigung der Oligonukleotide, unter Verwendung der OPC-Cartridges (Applied Biosystems), nach den Anweisungen des Herstellers.

Je 10 ng der Oligonukleotide HC572SY1.Seq und HC572SY2.SEQ wurden mit ca. 200 ng des mittels der Restriktionsenzyme EcoRI und HindIII geöffneten Vektors pUC8 für 16h bei 4°C ligiert und anschließend damit E.coli JM109 transformiert. Die Ausplattierung auf X-Gal/Ampicillin-haltigen Agarplatten sowie die Anzucht der Kolonien erfolgte wie oben beschrieben. Nach Isolierung der Plasmid-DNA erfolgte ein Restriktionsverdau mit PstI; diese Schnittstelle war durch das erste Oligonukleotidpaar neu eingeführt worden. Ein Klon, der eine Linearisierung zeigte, wurde für die weiteren Experimente verwendet. Die DNA dieses Klons wurde mit den Restriktionsenzymen PstI und EcoRI behandelt und mit je 10 ng der Oligonukleotide HC572SY3.SEQ und HC572SY4.SEQ ligiert. Transformation, Ausplattierung und Isolierung erfolgten wie oben beschrieben. Ein Klon, der nach Restriktionsverdau mit BsgI linarisiert worden war, wurde für den dritten Klonierungsschritt verwendet. Dieser wurde nach Restriktionsverdau mit BsgI und EcoRI mit den Oligonukleotiden HC572SY5.SEQ und HC572SY6.SEQ wie oben beschrieben durchgeführt. Ein Klon, der mit Hilfe von AvaI linarisiert worden war, wurde mit pUC8/UL57/2 bezeichnet und für die weiteren Arbeiten verwendet.

**Beipiel 9**

Klonierung der Fragmente UL57/1-3 in den Expressionsvektor pGEX-3X

Um die Expression der Antigenfragmente UL57/1-3 in Fusion mit dem heterologen Protein Glutathion-S-transferase (GST) zu gewährleisten, erfolgt die Umklonierung in den Expressionsvektor pGEX-3X (Smith, D. B., and K. S. Johnson. 1988. Single-step purification of polypeptides expressed in Escherichia coli as fusions with glutathione-S-transferase. Gene 67, 31-40). Dieser Vektor besitzt eine BamHI-Schnittstelle am 3' Ende des für GST kodierenden Gens. Der Leserahmen der BamHI-Schnittstelle stimmt mit denen der kodierenden Fragmente der Klone pUC8/UL57/1-3 überein. 100 mg der mittels EcorRI- und BamHI-Restriktionsverdau aus pUC8/UL57/1, pUC8/UL57/2 und pUC8/UL57/3 freigesetzten DNA-Fragmente wurden mit 200 ng des mit den gleichen Restriktionsenzymen geöffneten Vektors pGEX-3X für 16 h bei 4°C ligiert. Danach folgt eine Transformation mit E. coli JM109 und eine Ausplattierung auf Ampicillin-haltigen Platten. Isolierte Kolonien wurden in 3 ml LB-Medium mit Ampicillin überimpft und 16 h unter Schütteln bei 37°C inkubiert. Nach Isolierung der Plasmid-DNA erfolgte ein BamHI/EcoRI-Restriktionsverdau. Klone mit Inserts der zu erwartenden Größe wurden mit pGEX-3/UL57/1, pGEX-3/UL57/2 und pGEX-3/UL57/3 bezeichnet und für die Expression der UL57-Fragmente als GST-Fusionsproteine verwendet.

**Beispiel 10**

Klonierung der autologen Fusionsproteine 52/3 57/3 und 52/3 57/3 150/7/2

a) Ausgehend vom Klon pUC8/PCC52/3 - das klonierte Fragment kodiert für AS 297-433 von p52 - erfolgte eine PCR-Amplifikation mit Hilfe der Primer PCC525.seq und PCC526.seq und die spätere Klonierung des amplifizierten Fragments wie oben beschrieben. Aufgrund der Überhänge der beiden hier verwendeten Primer besitzt das amplifizierte Fragment am 5'-Ende eine BamHI und am 3'-Ende jeweils eine BglII- und EcoRI-Schnittstelle. Der entsprechende Klon wurde als pUC8/PCC52/3F bezeichnet.

b) Mit DNA des Klons pUC8/UL57/3 erfolgte ein Restriktionsverdau mit den Restriktionsenzymen XbaI und EcoRI. 200 ng des so geöffneten Vektors wurden mit je 10 ng der beiden Oligonukleotide UL57F3.SEQ und UL57F4.SEQ ligiert und anschließend damit E.coli JM109 wie oben beschrieben transformiert. Isolierte Kolonien wurden wie oben beschrieben in LB-Medium angeimpft und inkubiert. Nach Isolierung der Plasmid-DNA erfolgte ein Restriktionsverdau mit BglII, mit anschließender Agarosegel-Elektrophorese. Ein Klon, der eine Linarisierung als Nachweis der Integration der beiden Oligonukleotide zeigte, wurde mit pUC8/UL57/3F bezeichnet und für die weiteren Klonierungsarbeiten verwendet.

c) Mit DNA des Klons pUC8/UL57/3F erfolgte ein Restriktionsverdau mit BamHI und EcoRI. Das dabei freigesetzte Fragment von ca. 200 bp wurde mit Hilfe einer Agarose-Elektrophorese isoliert, und wie oben beschrieben aus dem herausgeschnittenen Gelstück eluiert. Ca. 50 ng dieses DNA-Fragmentes wurden dann mit 200 ng des mit BglII und EcoRI geöffneten und ebenfalls mittels Elektrophorese gereinigten Vektors pUC8PCC52/3F ligiert. Daran schloß sich eine Transformation mit E.coli JM109 und eine Ausplattierung auf Ampicillin-enthaltenden Agarplatten an. Isolierte Kolonien wurden in 3 ml LB-Medium mit Ampicillin überimpft und 12-16 h unter Schütteln bei 37°C inkubiert. Nach Isolierung der Plasmid-DNA erfolgte ein BamHI/EcoRI-Restriktionsverdau, mit anschließender Elektrophorese im Agarosegel. Ein Klon mit einem Insert der erwarteten Größe wurde als pUC8/52/3 UL57/3 bezeichnet und für die weiteren Experimente verwendet. Bei dieser Vorgehensweise wird ausgenutzt, daß die Erkennungssequenzen von BamHI und BglII die gleichen Überhänge besitzen, wodurch eine Ligation ermöglicht wird. Beide Schnittstellen gehen jedoch nach erfolgter Ligation verloren. Der Übergang von 52/3F zu UL57/3 ist so gewählt, daß eine Translation im gleichen Leserahmen möglich ist.

d) Mit DNA des Klons pUC8/PCC150/7/2 - dieser Klon enthält ein für die Aminosäuren 994-1048 des HCMV-Antigens pp150 codierendes Teilfragment - erfolgt ein Restriktionsverdau mit BamHI und EcoRI. Das so freigesetzte Fragment von ca.170 bp wird wie oben beschrieben elektrophoretisch gereinigt. 50 ng des gereinigten Fragments werden mit 200 ng des mit BglII und EcoRI behandelten Vektors pUC8/52/3 57/3 bei 4°C für 16h ligiert und damit E.coli JM109 transformiert. Die Ausplattierung erfolgte auf ampicillinhaltigen Agarplatten. Isolierte Kolonien wurden in 3 ml LB-Medium mit Ampicillin überimpft und 12-16 h unter Schütteln bei 37°C inkubiert. Nach Isolierung der Plasmid-DNA erfolgte ein BamHI/EcoRI Retriktionsverdau, mit anschließender Elektrophorese im Agarosegel. Ein Klon mit einem Insert der erwarteten Größe wurde mit pUC8/52/3 57/3 150/7/2 bezeichnet und für die weiteren Arbeiten verwendet.

e) Um die Expression des autologen Fusionsproteins zu gewährleisten, erfolgte die Umklonierung der entsprechenden DNA-Fragmente in den Vektor pET5c, der den starken T7-Promotor des Gens 10 des Bakteriophagen T7 besitzt. Dahinter besitzt dieser Vektor eine Ribosomenbindungsstelle und ein Startcodon in entsprechendem Abstand. Hinter dem Startcodon liegt ein Leserahmen von 11 Aminosäuren des Gens 10 des Bakteriophagen T7 und eine BamHI- und eine EcoRI-Schnittstelle. Das Leseraster der BamHI-Schnittstelle stimmt mit der der oben beschriebenen autologen Fusionsproteine überein. 100 ng der mittels EcoRI- und BamHI-Restriktionsverdau aus pUC8/52/3 UL57/3 und pUC8/52/3 57/3 150/7/2 freigesetzten DNA-Fragmente wurden mit 200 ng des mit den gleichen Restriktionsenzymen geöffneten Vektors für 16h bei 4°C ligiert. Danach folgte eine Transformation mit E.coli JM109 und eine Ausplattierung auf Ampicillin-haltigen Platten. Isolierte Kolonien wurden in 3 ml LB-Medium mit Ampicillin überimpft und 16 h unter Schütteln bei 37°C inkubiert. Nach Isolierung der Plasmid-DNA erfolgte ein BamHI/EcoRI-Restriktionsverdau. Klone mit Inserts der zu erwartenden Größe wurde als pET5a/52/3 UL57/3 bzw. pET5c/52/3 57/3 150/7/2 bezeichnet. Um die Expression des rekombinanten Proteins zu gewährleisten, wurde DNA der Expressionsklone in den chloramphenicolresistenten Expressionsstamm BL21(DE3)pLysS transformiert. Jeweils einer der resultierenden Klone wurde für die weiteren Arbeiten verwendet.

**Beispiel 11**

Chemische Synthese des Peptids UL57/3P und seine Reinigung

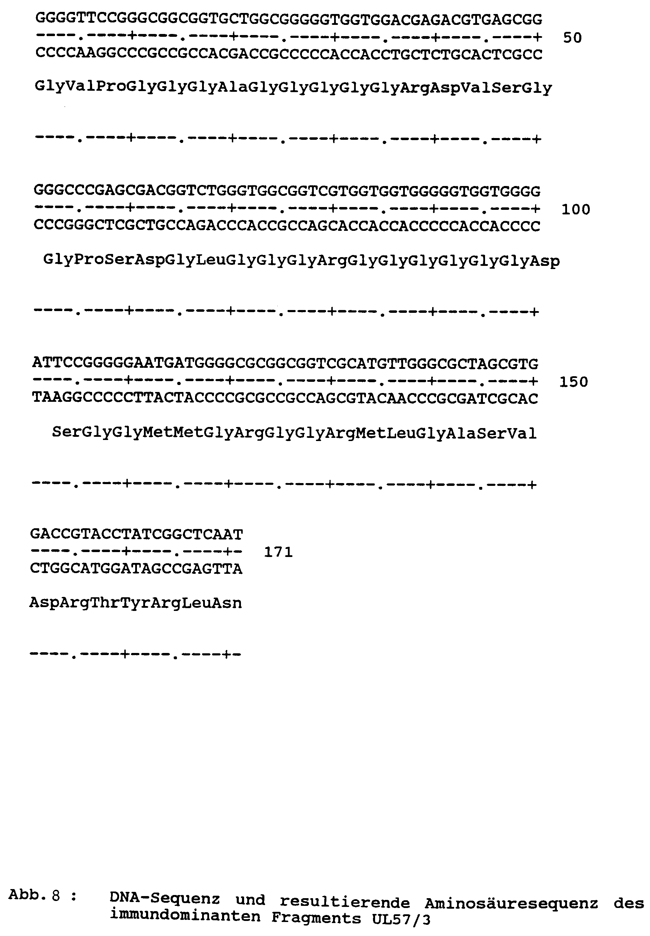

Die Synthese des Peptids UL57/3P erfolgte entsprechend der in Abb. 8 festgehaltenen Aminosäuresequenz mit einem Millipore 9050 continuous flow peptide synthesizer (Millipore Corp., Milford, MA, USA), unter Verwendung der 9-Fluorenylmethoxycarbonyl(Fmoc)-Chemie. Die Synthese erfolgte mit 1000 mg des Trägermaterials (PEG-PS resin) und 0.8 mmol des jeweiligen, aktivierten Aminosäureesters. Abspaltung vom Trägermaterial und Schutzgruppenentfernung erfolgte in einer 12stündigen Inkubation, in einem Gemisch aus 88 % Trifluoressigsäure, 5 % Phenol (flüssig), 2 % Triisopropylsilan und 5 % aqua dest. Freies Peptid wurde mehrfach mit eiskaltem Äther präzipitiert und anschließend im Vakuum getrocknet. Die Reinigung erfolgte mittels präperativer Reversed-Phase HPLC an einer C4-Säule (25 x 100 mm, 15 µm, 300A, Delta-Pak, Waters, Millipore Corp.) unter Verwendung eines Gradienten von 0-60 % Acetonitril in 0,1 % TFA). Die gesammelten Fraktionen wurden lyophilisiert und mittels Reversed-Phase HPLC und SDS-PAG-Elektrophorese analysiert. Fraktionen, die reines Peptid enthielten, wurden für die Evaluierung im ELISA verwendet.

**Beispiel 12**

Anzucht, Expression und Aufreinigung von UL57/1-GST, UL57/2-GST und UL57/3-GST

Mit einer isolierten Kolonie auf einer Agarplatte der Klone pGEX-3/UL57/1-3 (LB/AMP Medium) wurden 150 ml LB/AMP-Medium angeimpft. Die Kultivierung erfolgte in einem 1 l EMK, bei 37°, im Rundschüttler bei 160 Upm für 16 h.

Die Anzucht der 3 l Kultur erfolgte in 6 Parallelansätzen zu je 0.5 l in 2 l EMK mit Schikanen. Das Medium (LB/,AMP) war auf 37° vortemperiert. Die Kolben wurden inokuliert mit je 20 ml der Vorkultur (1:26) und inkubiert bei 37°C und 160 Upm im Rundschüttler. Das Wachstum wurde kontinuierlich verfolgt durch Messung der Absorption A bei 600 nm. Bei einer A (600 nm) von 0.7 wurde induziert durch Zugabe von IPTG (Endkonz. 1 mM).

Die Ernte erfolgte 4 h nach Induktion durch Zentrifugation (6x1 l-Becher, 4000 g, 0-4°C, 30 min). Die gut abgetropften Bakterienpellets wurden in 200 ml eiskaltem PBS resuspendiert und erneut zentrifugiert (2x250ml-Becher, 5000 g, 0-4°, 10 min). Die gut abgetropften Pellets wurden eingefroren und gelagert bei -20 bis -30°C.

Nach Auftauen der Bakterienpellets wurden diese in 80 ml Basis-Puffer (Tris-HCl/20mM/pH7.5) resuspendiert und anschließend homogenisiert (Teflon/Glas-Potter-Homogenisator). Unter Rühren bei Raumtemperatur wurden folgende Additive zugegeben: NP-40 (0.1 %), PMSF (0.1 mM), Pefabloc (0.1 mM), EDTA (50 mM) und Lysozym (100 mg). Das Gesamtvolumen betrug 100 ml. Der Ansatz wurde bei Raumtemperatur stark gerührt und nach 60 min auf Eis gestellt. Alle weiteren Schritte erfolgten auf Eis bzw. gekühlt. Nach der Inkubation wurde Glycerin (10 %) und 2-Mercaptoethanol (14 mM) zugegeben und vermischt. Das Volumen wurde mit Basis-Puffer auf 140 ml eingestellt. Der Lyseansatz wurde anschließend sonifiziert (20 kHz, pulsierend, 5 min, Titanrüssel 3/4"), gefolgt von maschinellem Pottern (Teflon/Glas-Potter-Homogenisator, 1000 UpM, 6 Stöße). Bei den Klonen pGEX-3/UL57/1 und pGEX/UL57/2 wurde solubilisiertes Material durch Zentrifugation abgetrennt (1 x 250 ml-Becher, 10000 g, 10 min, 0-4°C) und verworfen.

Beim Klon pGEX-3/UL57/3 wurde nichtsolubilisiertes Material durch Zentrifugation abgetrennt (4 x 50 ml-Becher, 40000 g, 30 min, 0-4°C) und verworfen.

Das nach der Lyse nicht solubilisierte Material (Pellet) des Klons pGEX-3/UL57/1 wurde in 50 ml Waschpuffer 1 (Tris-HCl / 100 mM / pH9 / 10 % Glycerin / 0.5 % NP-40 / 14 mM 2-Mercaptoethanol) vorhomogenisiert (Teflon/Glas-Potter-Homogenisator) und sonifiziert (20 kHz, pulsierend, 5 min, Titanrüssel 3/4"). Solubilisiertes Material wurde durch Zentrifugation abgetrennt (2 x 50 ml-Becher, 33000 g, 20 min, 0-4°C) und verworfen.

Das nicht solubilisierte Material (Pellets) wurde in 30 ml Waschpuffer 2 (Glycin-HCl / 100 mM / pH3 / 14 mM 2-Mercaptoethanol) vorhomogenisiert (Teflon/Glas-Potter-Homogenisator) und sonifiziert (20 kHz, pulsierend, 5 min, Titanrüssel 3/4"). Solubilisiertes Material wurde durch Zentrifugation abgetrennt (1 x 50 ml-Becher, 40000 g, 20 min, 0-4°C) und verworfen.

Das nicht solubilisierte Material (Pellet) wurde in 30 ml Waschpuffer 3 (Tris-HCl / 20 mM / pH9 / 4 M Harnstoff / 14 mM 2-Mercaptoethanol) vorhomogenisiert (Teflon/Glas-Potter-Homogenisator) und sonifiziert (20 kHz, pulsierend, 5 min, Titanrüssel 3/4"). Solubilisiertes Material wurde durch Zentrifugation abgetrennt (1 x 50 ml-Becher, 40000 g, 30 min, 0-4°C) und verworfen.

Das nach dem 3. Wasch-Schritt unlösliche Material (Pellet) wurde in 30 ml Solubilisierungspuffer (Tris-HCl / 20 mM / pH9 / 8 M Harnstoff / 14 mM 2-Mercaptoethanol) vorhomogenisiert (Teflon/Glas-Potter-Homogenisator) und sonifiziert (20 kHz, pulsierend, 5 min, Titanrüssel 3/4"). Weiterhin unlösliches Material wurde durch Zentrifugation abgetrennt (1 x 50 ml-Becher, 40000 g, 30 min, 8-10°C) und verworfen.

Das solubilisierte Protein wurde über SQ-Sepharose (HiLoad) chromatographiert. Dabei wurde eine Säule der Dimension 1.6 x 10 cm (20 ml) verwendet. A (280 nm) und Leitfähigkeit wurden kontinuierlich registriert. Der Fluß betrug 5 ml/min, Säulenpuffer = Solubilisierungspuffer. Nach Probenauftrag, gefolgt von 50 ml Säulenpuffer, wurde ein zunächst flacher, linearer NaCl-Gradient (dC/dV = 2.5 mM/ml, bis 500 mM) in Säulenpuffer, gefolgt von einem steilen, linearen NaCl-Gradient (dC/dV= 10mM/ml, bis 1000 mM) angelegt. Das Eluat wurde in 10 ml-Fraktionen zerlegt und eingefroren. Die Antigen-enthaltenden Fraktionen fanden sich im Bereich des flachen Gradienten. Der Nachweis erfolgte durch SDS-PA-Disc-Elektrophorese, mit anschließender Coomassie-Färbung.

Die Fraktionen, die nachweislich reines Antigen enthielten (Coomassie-Gel), wurden aufgetaut, vereint und mit gleichem Volumen an Renaturierungspuffer (Tris-HCl / 100 mM / pH9 / 10 % Glycerin / 14 mM 2-Mercaptoethanol) verdünnt (Erniedrigung der Harnstoffkonzentration auf 4 M). Alle weiteren Schritte erfolgten in einer Ultrafiltrationsrührzelle (Membran = Omega 50) unter Eiskühlung und Stickstoff-Atmosphäre. Die Proteinlösung wurde zunächst auf ca. 30 ml konzentriert und mit Renaturierungspuffer langsam auf das doppelte verdünnt. Weitere Konzentrierungen erfolgten jeweils auf die Hälfte des Ausgangsvolumens, gefolgt von Verdünnungen im Verhältnis 1:1 mit Renaturierungspuffer. Dabei wurde die Harnstoffkonzentration in Stufen abgesenkt: 4 M - 2 M - 1 M - 0.5 M - 0.25 M.

Die Endkonzentration an Harnstoff betrug maximal 0.25 M. Das Retentat wurde der Kammer entnommen und aliquotiert eingefroren und gelagert bei -60 bis -80°C.

Das nach der Lyse nicht solubilisierte Material (Pellet) des Klons pGEX-3/UL57/2 wurde in 50 ml Waschpuffer 1 (Tris-HCl / 100 mM / pH9 / 10 % Glycerin / 0.5 % NP-40 / 14 mM 2-Mercaptoethanol) vorhomogenisiert (Teflon/Glas-Potter-Homogenisator) und sonifiziert (20 kHz, pulsierend, 5 min, Titanrüssel 3/4"). Solubilisiertes Material wurde durch Zentrifugation abgetrennt (2 x 50 ml-Becher, 33000 g, 20 min, 0-4°C) und verworfen.

Das nicht solubilisierte Material (Pellets) wurde in 30 ml Waschpuffer 2 (Glycin-HCl / 100 mM / pH3 / 14 mM 2-Mercaptoethanol) vorhomogenisiert (Teflon/Glas-Potter-Homogenisator) und sonifiziert (20 kHz, pulsierend, 5 min, Titanrüssel 3/4"). Solubilisiertes Material wurde durch Zentrifugation abgetrennt (1 x 50 ml-Becher, 40000 g, 20 min, 0-4°C) und verworfen.

Das nicht solubilisierte Material (Pellet) wurde in 30 ml Waschpuffer 3 (Tris-HCl / 20 mM / pH9 / 4 M Harnstoff / 14 mM 2-Mercaptoethanol) vorhomogenisiert (Teflon/Glas-Potter-Homogenisator) und sonifiziert (20 kHz, pulsierend, 5 min, Titanrüssel 3/4"). Solubilisiertes Material wurde durch Zentrifugation abgetrennt (1 x 50 ml-Becher, 40000 g, 30 min, 0-4°C) und verworfen.

Das nach dem 3. Wasch-Schritt unlösliche Material (Pellet) wurde in 30 ml Solubilisierungspuffer (Tris-HCl / 20 mM / pH9 / 8 M Harnstoff / 14 mM 2-Mercaptoethanol) vorhomogenisiert (Teflon/Glas-Potter-Homogenisator) und sonifiziert (20 kHz, pulsierend, 5 min, Titanrüssel 3/4"). Weiterhin unlösliches Material wurde durch Zentrifugation abgetrennt (1 x 50 ml-Becher, 40000 g, 30 min, 8-10°C) und verworfen.

Das solubilisierte Fusionsprotein wurde mit gleichem Volumen an Renaturierungspuffer verdünnt (Erniedrigung der Harnstoffkonzentration auf 4 M). Alle weiteren Schritte erfolgten in einer Ultrafiltrationsrührzelle (Membran = Omega 50) unter Eiskühlung und Stickstoff-Atmosphäre. Die Proteinlösung wurde jeweils auf die Hälfte des Ausgangsvolumens konzentriert und wieder mit Renaturierungspuffer langsam verdünnt. Dabei wurde die Harnstoffkonzentration in Stufen abgesenkt: 4 M - 2 M - 1 M - 0.5 M - 0.25 M. Die Endkonzentration an Harnstoff betrug maximal 0.25 M. Das Retentat wurde der Kammer entnommen und aliqotiert eingefroren und gelagert bei -60 bis -80°C.

Das lösliche Protein des Klons pGEX-3/UL57/3 wurde über GSH-Sepharose-4B (Pharmacia) chromatographiert. Dabei wurde eine Säule der Dimension 2.6 x 10 cm (50 ml) verwendet. A (280 nm) wurde kontinuierlich registriert.

Der Fluß betrug 2 ml/min. Säulenpuffer = Tris-HCl / 20 mM / pH7.5 / 1.4 mM Mercaptoethanol. Nach erfolgtem Probenauftrag wurde solange mit Säulenpuffer nachgespült, bis der Schreiber die Basislinie wieder erreicht hatte. Das GST-Protein wurde mit 5 mM Glutathion (GSHred.) in Säulenpuffer eluiert. Das GSH wurde unmittelbar vor der Verwendung gelöst. Das GSH-Eluat (vom Schreiber registrierter Absorptionspeak) wurde gesammelt und aliquotiert eingefroren und gelagert bei -60 bis -80°C.

**Beispiel 13**

Anzucht, Expression und Aufreinigung des autologen Fusionsproteins 52/3 57/3

Ausgehend von einer Plattenkolonie (LB/CA,AMP) des Klons pET5c 52/3 57/3 wurde eine 15 ml Kultur (LB/CA, AMP) bei 37°C im Rundschüttler bis zu einer A (600 nm) = 1.8-2.0 angezogen. Die Kultur wurde dann vermischt mit Glycerin (87 %), bis zu einer Endkonzentration von 15 % (v/v). Die Kultur wurde in 0.1 ml-Portionen eingefroren und gelagert bei -60 bis -80°C.

Ein gefrorenes Aliquot der Vorkultur wurde rasch aufgetaut und in 150 ml LB/CA, AMP-Medium einpipettiert. Die Kultivierung erfolgte in einem 1 l EMK, bei 28°C, im Rundschüttler bei 100 Upm, für 16 h.

Die Anzucht der 6 l Kultur erfolgte in 12 Parallelansätzen zu je 0.5 l in 2 l EMK mit Schikanen. Das Medium (LB/CA, AMP) war auf 37°C vortemperiert. Die Kolben wurden inokuliert mit je 10 ml der Vorkultur (1:51) und inkubiert bei 37°C und 160 Upm im Rundschüttler. Das Wachstum wurde kontinuierlich verfolgt, durch Messung von A (600 nm). Bei einer A (600 nm) von 0.6 wurde induziert, durch Zugabe von IPTG (Endkonz. 1 mM).

Die Ernte erfolgte 3 h nach Induktion durch Zentrifugation (6 x 1 l-Becher, 4000 g, 0-4°C, 30 min). Die gut abgetropften Bakterienpellets wurden in 200 ml eiskaltem PBS resuspendiert und erneut zentrifugiert (2 x 250 ml-Becher, 5000 g, 0-4°C, 10 min). Die gut abgetropften Pellets wurden eingefroren und gelagert bei -20 bis -30°C.

Die eingefrorenen Bakterienpellets wurden aufgetaut und resuspendiert in 160 ml Basis-Puffer (Tris-HCl / 20 mM / pH7.5) und anschließend mit einem (Teflon/Glas-Potter-Homogenisator) homogenisiert. Unter Rühren bei Raumtemperatur wurden folgende Additive zugegeben: NP-40 (0.05 %), PMSF (0.2 mM), Pefabloc (0.2 mM), EDTA (50 mM) und Lysozym (50 mg). Das Gesamtvolumen betrug 200 ml. Der Ansatz wurde nach Lysozymzugabe sofort auf Eis gestellt. Alle weiteren Schritte erfolgten auf Eis bzw. gekühlt. Nach der Inkubation wurde Glycerin (10 %) und 2-Mercaptoethanol (14 mM) zugegeben und vermischt. Das Volumen wurde mit Basis-Puffer auf 280 ml eingestellt. Der Lyseansatz wurde anschließend sonifiziert (20 kHz, pulsierend, 5 min, Titanrüssel 3/4"). Nicht solubilisiertes Material wurde durch Zentrifugation entfernt (2 x 250 ml-Becher, 27000 g, 30 min, 0-4°C). Die Pellets wurden verworfen.

Dem Überstand wurde unter Rühren im Eisbad festes, fein gemörsertes Ammoniumsulfat langsam zugegeben (innerhalb 15 min) bis zu einer Konzentration von 30 % Sättigung. Es wurde noch 15 min weiter gerührt. Anschließend wurde zentrifugiert (2 x 250 ml-Becher, 27000 g, 30 min, 0-4°C). Die Pellets wurden verworfen. Der Überstand wurde erneut mit Ammoniumsulfat versetzt, bis zu einer Konzentration von 43 % Sättigung und wie vorher zentrifugiert. Der Überstand wurde verworfen. Die Pellets wurden resuspendiert in 25 ml Tris-HCl / 20 mM / pH8.5, der zusätzlich 2-Mercaptoethanol (14 mM), Pefabloc (0.1 mM) und Glycerin (10 %) enthielt, eingeforen und über Nacht gelagert bei -20° bis -30°C.

Die mit Ammoniumsulfat (30-43 %) fraktionierte Protein-Lösung wurde aufgetaut, und präzipitiertes Protein wurde entfernt durch Zentrifugation (1 x 50 ml-Becher, 40000 g, 30 min, 0-4°C). Der Überstand wurde über eine Sephadex G-25 (coarse) Säule (Volumen mindestens 200 ml) chromatographiert, wobei A (280 nm) und die Leitfähigkeit im Durchfluß gemessen wurden. Säulenpuffer = Tris-HCl / 20 mM / pH8.5 mit 2-Mercaptoethanol (1.4 mM), Pefabloc (0.02 mM) und Glycerin (10 %). Das Protein im Ausschlußvolumen wurde vollständig gesammelt und direkt weiter chromatographiert.

Das Sephadex G-25 Eluat wurde über SP-Sepharose (Fast Flow) chromatographiert. Dabei wurde eine Säule der Dimension 2.6 x 12 cm (60 ml) verwendet. A (280 nm) und Leitfähigkeit wurden kontinuierlich registriert. Der Fluß betrug 5 ml/min. Säulenpuffer = Tris-HCl / 20 mM / pH8.5 mit 2-Mercaptoethanol (1.4 mM), Pefabloc (0.02 mM) und Glycerin (10 %). Nach Probenauftrag, gefolgt von 100 ml Säulenpuffer, wurde ein linearer NaCl-Gradient (dC/dV = 1.2 mM/ml, bis 500 mM ) in Säulenpuffer angelegt. Das Eluat wurde in 10 ml-Fraktionen zerlegt und eingefroren. Die Antigen-enthaltenden Fraktionen fanden sich im Bereich zwischen 200-300 mM NaCl. Der Nachweis erfolgte durch SDS-PA-Disc-Elektrophorese mit anschließender Coomassie-Färbung.

Die Fraktionen, die nachweislich intaktes Antigen (28.8/27.5 kD Banden) enthielten (Coomassie-Gel), wurden aufgetaut, vereint, mit Glycerin versetzt (20 %) und mittels Ultrafiltration (Rührzelle, Eisbad, Membran = Omega 30) konzentriert bis zu einem Endvolumen von ca. 5 ml. Das Retentat wurde der Kammer entnommen und anschließend chromatographiert.

Der konzentrierte SP-Sepharose-Pool wurde über Superdex 75 (prep grade) chromatographiert. Dabei wurde eine HiLoad-Säule der Dimension 2.6 x 60 cm (300 ml) verwendet. A (280 nm) und Leitfähigkeit wurden kontinuierlich registriert. Der Fluß betrug 2 ml/min. Säulenpuffer = Basispuffer mit NaCl (0.5 M), Glycerin (20 %), 2-Mercaptoethanol (1.4 mM), EDTA (1 mM), PMSF (0.1 mM) und Pefabloc (0.02 mM). Das Eluat wurde in 5 ml-Fraktionen zerlegt und eingefroren. Die Antigenenthaltenden Fraktionen fanden sich im 2. Absorptionspeak. Der Nachweis erfolgte durch SDS-PA-Disc-Elektrophorese mit anschließender Coomassie-Färbung.

Die Fraktionen, die nachweislich intaktes und reines Antigen enthielten (Coomassie-Gel), wurden aufgetaut, vereint und mittels Ultrafiltration (Rührzelle, Eisbad, Membran = Omega 30) konzentriert bis zu einer Endkonzentration von mindestens 2 mg/ml. Das Retentat wurde der Kammer entnommen und aliqotiert eingefroren und gelagert bei -60 bis -80°C.

**Beispiel 14**

Anzucht, Expression und Aufreinigung des autologen Fusionsproteins 52/3 57/3 150/7/2

Anzucht und Lyse erfolgte, wie beim autologen Fusionsprotein 52/3 57/3 beschrieben.

Dem nach der Lyse anfallenden Überstand wurde unter Rühren im Eisbad festes, fein gemörsertes Ammoniumsulfat langsam zugegeben (innerhalb 15min) bis zu einer Konzentration von 30% Sättigung. Es wurde noch 15min weiter gerührt. Anschließend wurde zentrifugiert (2x250ml-Becher, 27000g, 30min, 0-4°). Die Pellets wurden verworfen. Der Überstand wurde erneut mit Ammoniumsulfat versetzt bis zu einer Konzentration von 38% Sättigung und wie vorher zentrifugiert. Der Überstand wurde verworfen. Die Pellets wurden resuspendiert in 25 ml Tris-HCl/20mM/pH9-Puffer, der zusätzlich 2-Mercaptoethanol (14mM), Pefabloc (0.1 mM) und Glycerin (10%) enthielt, eingefroren und über Nacht gelagert bei -20° bis -30°C.

Die mit Ammoniumsulfat (30-38%) fraktionierte Protein-Lösung wurde aufgetaut und präzipitiertes Protein wurde entfernt durch Zentrifugation (1x50 ml-Becher, 40000g, 30min, 0-4°C). Der Überstand wurde über eine Sephadex G-25 (coarse) Säule (Volumen mindestens 200 ml) chromatographiert, wobei A(280nm) und die Leitfähigkeit im Durchfluß gemessen wurden. Säulenpuffer = Tris-HCl/20mM/pH9 mit 2-Mercaptoethanol (1.4 mM), Pefabloc (0.02 mM) und Glycerin (10%). Das Protein im Ausschlußvolumen wurde vollständig gesammelt und direkt weiter chromatographiert.

Das Sephadex G-25 Eluat wurde über SP-Sepharose (Fast Flow) chromatographiert. Dabei wurde eine Säule der Dimension 2.6 x 12 cm (60 ml) verwendet. A (280nm) und Leitfähigkeit wurden kontinuierlich registriert. Der Fluß betrug 5 ml/min. Säulenpuffer = Tris-HCl/20mM/pH9 mit 2-Mercaptoethanol (1.4 mM), Pefabloc (0.02 mM) und Glycerin (10%). Nach Probenauftrag, gefolgt von 100 ml Säulenpuffer, wurde ein linearer NaCl-Gradient (dC/dV= 1.7 mM/ml, bis 500 mM ) in Säulenpuffer angelegt. Das Eluat wurde in 10 ml-Fraktionen zerlegt und eingefroren. Die Antigen-enthaltenden Fraktionen fanden sich im Bereich zwischen 150-300 mM NaCl. Der Nachweis erfolgte durch SDS-PA-Disc-Elektrophorese mit anschließender Coomassie-Färbung.

Die Fraktionen, die nachweislich intaktes Antigen (33 kD Bande) enthielten (Coomassie-Gel), wurden aufgetaut, vereint, mit Glycerin versetzt (20%) und mittels Ultrafiltration (Rührzelle, Eisbad, Membran = Omega 30) konzentriert bis zu einem Endvolumen von ca. 5 ml. Das Retentat wurde der Kammer entnommen und anschließend chromatographiert.

Der konzentrierte SP-Sepharose-Pool wurde über Superdex 75 (prep grade) chromatographiert. Dabei wurde eine HiLoad-Säule der Dimension 2.6 x 60 cm (300 ml) verwendet. A(280nm) und Leitfähigkeit wurden kontinuierlich registriert. Der Fluß betrug 2 ml/min. Säulenpuffer = Basispuffer mit NaCl (0.5 M), Glycerin (20%), 2-Mercaptoethanol (1.4 mM), EDTA (1 mM), PMSF (0.1 mM) und Pefabloc (0.02 mM). Das Eluat wurde in 5 ml-Fraktionen zerlegt und eingefroren. Die Antigen-enthaltenden Fraktionen fanden sich im 1. Absorptionspeak (= größter peak). Der Nachweis erfolgte durch SDS-PA-Disc-Elektrophorese mit anschließender Coomassie-Färbung.

Die Fraktionen, die nachweislich intaktes und reines Antigen enthielten (Coomassie-Gel), wurden aufgetaut, vereint und mittels Ultrafiltration (Rührzelle, Eisbad, Membran = Omega 30) konzentriert bis zu einer Endkonzentration von mindestens 2 mg/ml. Das Retentat wurde der Kammer entnommen und aliqotiert eingefroren und gelagert bei -60 bis -80°C.

**Beispiel 15**

Evaluierung der rekombinanten Antigene im ELISA

Je 100 ng der gereinigten rekombinanten Antigene UL57/1, UL57/3 und 52/3 UL57/3 bzw. 200 ng der Antigene UL57/2 und 52/3 57/3 150/7/2 wurden in je 100 µl 0,01 M Carbonatpuffer pH 9,5 gelöst, in die Vertiefungen von Mikrotiterplatten (Polycorb, Nunc) gegeben und 16 h in einer geerdeten feuchten Kammer inkubiert. Nach Zugabe von 100 µl einer Kalbsserumenthaltenden Nachbeschichtungslösung wurde die Inkubation für 2 weitere Stunden fortgesetzt. Danach wurden die Platten entleert und sorgfältig ausgeschlagen. Anschließend wurden die Platten für die eigentliche Testdurchführung verwendet, oder nach Trocknung im Vakuumschrank und anschließendem Einschweißen in Folienschlauch bis zum späteren Gebrauch bei -20°C gelagert. Die Beschichtung des chemisch synthetisierten Peptids erfolgte mit 2 µg/100 µl wie oben beschrieben unter Verwendung von Maxisorb-ELISA-Platten (Nunc). Zur eigentlichen Testdurchführung wurden die Testnäpfchen der ELISA-Platten mit 100 µl 1:21 verdünntem Serum gefüllt, mit einer Plastikfolie abgeklebt und 1 h bei 40°C schwimmend in Wasserbad inkubiert. Nach dreimaligem Waschen im Biotest ELISA Washer II erfolgte die Inkubation mit 100 µl eines gegen humanes IgM- gerichteten, Peroxidase-markierten, monoklonalen Antikörpers aus der Maus (Janssen) für 30 min bei 40°C. Nach abermaligem Waschen wurden die gebundenen Antikörper durch eine Farbreaktion mit 1,2-Phenyldiamin als Chromogen sichtbar gemacht. Die optische Dichte der einzelnen Probe wurde bei 495 nm (Referenz: 620 nm) am Anthos HTII ELISA-Reader ermittelt. Alle OD-Werte größer als 0,3 wurden als positives Ergebnis gewertet.

Die Ergebnisse der so erhaltenen Evaluierungen sind in den Tabellen 3-5 dargestellt.

SEQUENZPROTOKOLL

(1) ALLGEMEINE INFORMATION:

    ANMELDER:
        (A) NAME: Biotest AG
        (B) STRASSE: Landsteinerstr. 5
        (C) ORT: Dreieich
        (E) LAND: Germany
        (F) POSTLEITZAHL: 63276

    ANMELDETITEL:
        Polypeptide und Fusionsproteine umfassend eine
        vom Cytomegalievirus stammende Aminosäuresequenz
        aus dem Leserahmen UL57 bzw. dem C-terminalen Be-
        reich des Tegumentproteins pp150, diese enthal-
        tende diagnostische Testkits, Verfahren zum Nach-
        weis von Antikörpern gegen Cytomegalievirus und
        DNA-Oligonucleotide

    ANZAHL DER SEQUENZEN: 27

    COMPUTER-LESBARE FORM:
        (A) DATENTRÄGER: Floppy Disk
        (B) COMPUTER: IBM PC compatible
        (C) BETRIEBSSYSTEM: PC-DOS/MS-DOS
        (D) SOFTWARE: PADAT Sequenzmodul Version 1.0

(2) INFORMATION ZU SEQ ID NO:  1:

    (i) SEQUENZ CHARAKTERISTIKA:
        (A) LÄNGE: 57 Aminosäuren
        (B) ART: Aminosäure
        (C) STRANGFORM: Einzel
        (D) TOPOLOGIE: linear

    (ii) ART DES MOLEKÜLS: Protein

    (v) ART DES FRAGMENTS: inneres

    (xi) SEQUENZBESCHREIBUNG: SEQ ID NO:  1: (Seite 5 der Beschreibung)

```
    Gly Val Pro Gly Gly Gly Ala Gly Gly Gly Gly Gly Arg Asp
Val Ser
     1               5                        10
 15

    Gly Gly Pro Ser Asp Gly Leu Gly Gly Gly Arg Gly Gly Gly
Gly Gly
               20                      25                    30

    Gly Asp Ser Gly Gly Met Met Gly Arg Gly Gly Arg Met Leu
Gly Ala
               35                      40                    45

    Ser Val Asp Arg Thr Tyr Arg Leu Asn
         50                    55
```

(2) INFORMATION ZU SEQ ID NO:  2: (Fig. 1, erste Sequenz)

    (i) SEQUENZ CHARAKTERISTIKA:
        (A) LÄNGE: 27 Basenpaare
        (B) ART: DNA
        (C) STRANGFORM: Einzel
        (D) TOPOLOGIE: linear

    (ii) ART DES MOLEKÜLS: Primer-DNA

    (xi) SEQUENZBESCHREIBUNG: SEQ ID NO:  2:

```
GAATTCTATT CCTCCGTGTT CTTAATC
27
```

(2) INFORMATION ZU SEQ ID NO:  3: (Fig. 1, zweite
Sequenz)

   (i) SEQUENZ CHARAKTERISTIKA:
      (A) LÄNGE: 26 Basenpaare
      (B) ART: DNA
      (C) STRANGFORM: Einzel
      (D) TOPOLOGIE: linear

  (ii) ART DES MOLEKÜLS: Primer-DNA

  (xi) SEQUENZBESCHREIBUNG: SEQ ID NO:  3:

CGGATCCTGA AGAGCACGAC GGGCAT
26

(2) INFORMATION ZU SEQ ID NO: 4: (Fig. 1, dritte
                                  Sequenz)

    (i) SEQUENZ CHARAKTERISTIKA:
        (A) LÄNGE: 35 Basenpaare
        (B) ART: DNA
        (C) STRANGFORM: Einzel
        (D) TOPOLOGIE: linear

    (ii) ART DES MOLEKÜLS: Primer-DNA


    (xi) SEQUENZBESCHREIBUNG: SEQ ID NO: 4:

GGATCCGCAT GCGTGGCAGC CTCTCTTCGC TGGCC
35


(2) INFORMATION ZU SEQ ID NO: 5: (Fig. 1, vierte
                                  Sequenz)

    (i) SEQUENZ CHARAKTERISTIKA:
        (A) LÄNGE: 30 Basenpaare
        (B) ART: DNA
        (C) STRANGFORM: Einzel
        (D) TOPOLOGIE: linear

    (ii) ART DES MOLEKÜLS: Primer-DNA


    (xi) SEQUENZBESCHREIBUNG: SEQ ID NO: 5:

GAATTCAGAT CTTGCCGCAC TTTTGCTTCT
30

(2) INFORMATION ZU SEQ ID NO: 6: (Fig. 2, oberer
                                    Strang)

    (i) SEQUENZ CHARAKTERISTIKA:
        (A) LÄNGE: 607 Basenpaare
        (B) ART: DNA
        (C) STRANGFORM: Einzel
        (D) TOPOLOGIE: linear

   (ii) ART DES MOLEKÜLS: Genom-DNA


   (xi) SEQUENZBESCHREIBUNG: SEQ ID NO: 6:

    GGATCCGCAT GCGTGGCAGC CTCTCTTCGC TGGCCAATGC CGGCGGTCTG
CATGACGACG        60

    GCCCGGGTCT GGATAACGAT CTCATGAACG AGCCCATGGG TCTCGGCGGT
CTGGGAGGAG        120

    GTGGCGGCGG TGGCGGCAAG AAGCACGACC GCGGTGGCGG CGGTGGTTCC
GGTACGCGGA        180

    AAATGAGTAG CGGTGGCGGC GGCGGTGATC ATGACCACGG TCTTTCCTCC
AAGGAAAAAT        240

    ACGAGCAGCA CAAGATCACC AGCTACCTGA CGTCCAAAGG TGGATCGGGC
GGCGGCGGAG        300

    GAGGAGGAGG CGGCGGTTTG GATCGCAACT CCGGCAATTA CTTCAACGAC
GCGAAAGAGG        360

    AGAGCGACAG CGAGGATTCT GTAACGTTCG AGTTCGTCCC TAACACCAAG
AAGCAAAAGT        420

    GCGGCAAGAT CCTGAAGAGC ACGACGGGCA TGAAAACGGT GGCTTTCGAC
CTATCGTCGC        480

    CCCAGAAGAG CGGTACGGGG CCGCAACCGG GTTCTGCCGG CATGGGGGGC
GCCAAAACGC        540

    CGTCGGACGC CGTGCAGAAC ATCCTCCAAA AGATCGAGAA GATTAAGAAC
ACGGAGGAAT        600

    AGAATTC
607

(2) INFORMATION ZU SEQ ID NO: 7: (Fig. 2, unterer
Strang)

   (i) SEQUENZ CHARAKTERISTIKA:
      (A) LÄNGE: 607 Basenpaare
      (B) ART: DNA
      (C) STRANGFORM: Einzel
      (D) TOPOLOGIE: linear

  (ii) ART DES MOLEKÜLS: Genom-DNA

  (xi) SEQUENZBESCHREIBUNG: SEQ ID NO: 7:

```
CCTAGGCGTA CGCACCGTCG GAGAGAAGCG ACCGGTTACG GCCGCCAGAC
GTACTGCTGC        60

CGGGCCCAGA CCTATTGCTA GAGTACTTGC TCGGGTACCC AGAGCCGCCA
GACCCTCCTC        120

CACCGCCGCC ACCGCCGTTC TTCGTGCTGG CGCCACCGCC GCCACCAAGG
CCATGCGCCT        180

TTTACTCATC GCCACCGCCG CCGCCACTAG TACTGGTGCC AGAAAGGAGG
TTCCTTTTTA        240

TGCTCGTCGT GTTCTAGTGG TCGATGGACT GCAGGTTTCC ACCTAGCCCG
CCGCCGCCTC        300

CTCCTCCTCC GCCGCCAAAC CTAGCGTTGA GGCCGTTAAT GAAGTTGCTG
CGCTTTCTCC        360

TCTCGCTGTC GCTCCTAAGA CATTGCAAGC TCAAGCAGGG ATTGTGGTTC
TTCGTTTTCA        420

CGCCGTTCTA GGACTTCTCG TGCTGCCCGT ACTTTTGCCA CCGAAAGCTG
GATAGCAGCG        480

GGGTCTTCTC GCCATGCCCC GGCGTTGGCC CAAGACGGCC GTACCCCCCG
CGGTTTTGCG        540

GCAGCCTGCG GCACGTCTTG TAGGAGGTTT TCTAGCTCTT CTAATTCTTG
TGCCTCCTTA        600

TCTTAAG
607
```

(2) INFORMATION ZU SEQ ID NO: 8: (Fig. 2,
Aminosäuresequenz)

   (i) SEQUENZ CHARAKTERISTIKA:
      (A) LÄNGE: 199 Aminosäuren
      (B) ART: Aminosäure
      (C) STRANGFORM: Einzel
      (D) TOPOLOGIE: linear

  (ii) ART DES MOLEKÜLS: Protein

  (v) ART DES FRAGMENTS: inneres

  (xi) SEQUENZBESCHREIBUNG: SEQ ID NO: 8:

```
Ile Arg Met Arg Gly Ser Leu Ser Ser Leu Ala Asn Ala Gly
Gly Leu
 1           5                   10
15

His Asp Asp Gly Pro Gly Leu Asp Asn Asp Leu Met Asn Glu
Pro Met
             20                  25                  30

Gly Leu Gly Gly Leu Gly Gly Gly Gly Gly Gly Gly Gly Lys
Lys His
             35                  40                  45

Asp Arg Gly Gly Gly Gly Gly Ser Gly Thr Arg Lys Met Ser
Ser Gly
     50                  55                  60

Gly Gly Gly Gly Asp His Asp His Gly Leu Ser Ser Lys Glu
Lys Tyr
     65                  70                  75
80

Glu Gln His Lys Ile Thr Ser Tyr Leu Thr Ser Lys Gly Gly
Ser Gly
                 85                  90
95

Gly Gly Gly Gly Gly Gly Gly Gly Gly Leu Asp Arg Asn Ser
Gly Asn
                 100                 105                 110

Tyr Phe Asn Asp Ala Lys Glu Glu Ser Asp Ser Glu Asp Ser
Val Thr
             115                 120                 125

Phe Glu Phe Val Pro Asn Thr Lys Lys Gln Lys Cys Gly Lys
Ile Leu
             130                 135                 140
```

```
        Lys Ser Thr Thr Gly Met Lys Thr Val Ala Phe Asp Leu Ser
    Ser Pro
        145                 150                 155
    160

        Gln Lys Ser Gly Thr Gly Pro Gln Pro Gly Ser Ala Gly Met
    Gly Gly
                        165                 170
    175

        Ala Lys Thr Pro Ser Asp Ala Val Gln Asn Ile Leu Gln Lys
    Ile Glu
                    180                 185                 190

        Lys Ile Lys Asn Thr Glu Glu
                195
```

(2) INFORMATION ZU SEQ ID NO: 9: (Fig. 7, erste Sequenz)

    (i) SEQUENZ CHARAKTERISTIKA:
        (A) LÄNGE: 33 Basenpaare
        (B) ART: DNA
        (C) STRANGFORM: Einzel
        (D) TOPOLOGIE: linear

    (ii) ART DES MOLEKÜLS: Primer-DNA

    (xi) SEQUENZBESCHREIBUNG: SEQ ID NO: 9:

GGATCCGCAT GCATCACGAC CGCCTGCTGG ACT
33

(2) INFORMATION ZU SEQ ID NO: 10: (Fig. 7, zweite Sequenz)

    (i) SEQUENZ CHARAKTERISTIKA:
        (A) LÄNGE: 29 Basenpaare
        (B) ART: DNA
        (C) STRANGFORM: Einzel
        (D) TOPOLOGIE: linear

    (ii) ART DES MOLEKÜLS: Primer-DNA

    (xi) SEQUENZBESCHREIBUNG: SEQ ID NO: 10:

GAATTCTTAG TTGTTGATAC CCGCATATT
29

(2) INFORMATION ZU SEQ ID NO: 11: (Fig. 7, dritte Sequenz)

    (i) SEQUENZ CHARAKTERISTIKA:
        (A) LÄNGE: 34 Basenpaare
        (B) ART: DNA
        (C) STRANGFORM: Einzel
        (D) TOPOLOGIE: linear

    (ii) ART DES MOLEKÜLS: Primer-DNA

    (xi) SEQUENZBESCHREIBUNG: SEQ ID NO: 11:

GGATCCGCAT GCATGGGGTT CCGGGCGGCG GTGC
34

(2) INFORMATION ZU SEQ ID NO: 12: (Fig. 7, vierte Sequenz)

    (i) SEQUENZ CHARAKTERISTIKA:
        (A) LÄNGE: 31 Basenpaare
        (B) ART: DNA
        (C) STRANGFORM: Einzel
        (D) TOPOLOGIE: linear

    (ii) ART DES MOLEKÜLS: Primer-DNA

    (xi) SEQUENZBESCHREIBUNG: SEQ ID NO: 12:

GAATTCTCTA GAATTGAGCC GATAGGTACG G
31

(2) INFORMATION ZU SEQ ID NO: 13: (Fig. 7, fünfte Sequenz)

    (i) SEQUENZ CHARAKTERISTIKA:
        (A) LÄNGE: 42 Basenpaare
        (B) ART: DNA
        (C) STRANGFORM: Einzel
        (D) TOPOLOGIE: linear

    (ii) ART DES MOLEKÜLS: Primer-DNA

    (xi) SEQUENZBESCHREIBUNG: SEQ ID NO: 13:

GATCCCCTCT AGAGACGCTC AGCGTCTTAC TGACGCTGCA GG
42

(2) INFORMATION ZU SEQ ID NO: 14: (Fig. 7, sechste
                                    Sequenz)

    (i) SEQUENZ CHARAKTERISTIKA:
       (A) LÄNGE: 42 Basenpaare
       (B) ART: DNA
       (C) STRANGFORM: Einzel
       (D) TOPOLOGIE: linear

   (ii) ART DES MOLEKÜLS: Primer-DNA

   (xi) SEQUENZBESCHREIBUNG: SEQ ID NO: 14:

AATCTCTGCA GCGTCAGTAA GACGCTGAGC GTCTCTAGAG GG
42

(2) INFORMATION ZU SEQ ID NO: 15: (Fig. 7, siebente
                                   Sequenz)

    (i) SEQUENZ CHARAKTERISTIKA:
        (A) LÄNGE: 72 Basenpaare
        (B) ART: DNA
        (C) STRANGFORM: Einzel
        (D) TOPOLOGIE: linear

    (ii) ART DES MOLEKÜLS: Primer-DNA


    (xi) SEQUENZBESCHREIBUNG: SEQ ID NO: 15:

    GGTGGTGAAG TTCATGACCT TTCTGCTCTT TTCGCTCCGT CTGGTGTTGG
TGCAGCTTCT          60

    GGTGTTGGTG GG
72


(2) INFORMATION ZU SEQ ID NO: 16: (Fig. 7, achte
                                   Sequenz)

    (i) SEQUENZ CHARAKTERISTIKA:
        (A) LÄNGE: 80 Basenpaare
        (B) ART: DNA
        (C) STRANGFORM: Einzel
        (D) TOPOLOGIE: linear

    (ii) ART DES MOLEKÜLS: Primer-DNA


    (xi) SEQUENZBESCHREIBUNG: SEQ ID NO: 16:

    AATCTCCACC AACACCAGAA GCTGCACCAA CACCAGACGG AGCGAAAAGA
GCAGAAAGGT          60

    CATGAACTTC ACCACCTGCA
80

(2) INFORMATION ZU SEQ ID NO: 17: (Fig. 7, neunte
                                    Sequenz)

   (i) SEQUENZ CHARAKTERISTIKA:
       (A) LÄNGE: 61 Basenpaare
       (B) ART: DNA
       (C) STRANGFORM: Einzel
       (D) TOPOLOGIE: linear

   (ii) ART DES MOLEKÜLS: Primer-DNA


   (xi) SEQUENZBESCHREIBUNG: SEQ ID NO: 17:

   TGGTGGTCTG CTTCTTGGTG AATCTGTTGC TGGTAACTCT ATCTGCTTCG
GTGTCCCGGG          60

      G
61

(2) INFORMATION ZU SEQ ID NO: 18: (Fig. 7, zehnte
                                    Sequenz)

    (i) SEQUENZ CHARAKTERISTIKA:
        (A) LÄNGE: 67 Basenpaare
        (B) ART: DNA
        (C) STRANGFORM: Einzel
        (D) TOPOLOGIE: linear

    (ii) ART DES MOLEKÜLS: Primer-DNA


    (xi) SEQUENZBESCHREIBUNG: SEQ ID NO: 18:

AATTCCCCGG GACACCGAAG CAGATAGAGT TACCAGCAAC AGATTCACCA
AGAAGCAGAC          60

CACCACC
67



(2) INFORMATION ZU SEQ ID NO: 19: (Fig. 8, oberer
                                    Strang)

    (i) SEQUENZ CHARAKTERISTIKA:
        (A) LÄNGE: 171 Basenpaare
        (B) ART: DNA
        (C) STRANGFORM: Einzel
        (D) TOPOLOGIE: linear

    (ii) ART DES MOLEKÜLS: Genom-DNA


    (xi) SEQUENZBESCHREIBUNG: SEQ ID NO: 19:

GGGGTTCCGG GCGGCGGTGC TGGCGGGGGT GGTGGACGAG ACGTGAGCGG
GGGCCCGAGC          60

GACGGTCTGG GTGGCGGTCG TGGTGGTGGG GGTGGTGGGG ATTCCGGGGG
AATGATGGGG          120

CGCGGCGGTC GCATGTTGGG CGCTAGCGTG GACCGTACCT ATCGGCTCAA T
171

(2) INFORMATION ZU SEQ ID NO: 20: (Fig. 8, unterer
                                        Strang)

  (i) SEQUENZ CHARAKTERISTIKA:
       (A) LÄNGE: 171 Basenpaare
       (B) ART: DNA
       (C) STRANGFORM: Einzel
       (D) TOPOLOGIE: linear

  (ii) ART DES MOLEKÜLS: Genom-DNA


  (xi) SEQUENZBESCHREIBUNG: SEQ ID NO: 20:

CCCCAAGGCC CGCCGCCACG ACCGCCCCCA CCACCTGCTC TGCACTCGCC
CCCGGGCTCG        60

CTGCCAGACC CACCGCCAGC ACCACCACCC CCACCACCCC TAAGGCCCCC
TTACTACCCC       120

GCGCCGCCAG CGTACAACCC GCGATCGCAC CTGGCATGGA TAGCCGAGTT A
171




(2) INFORMATION ZU SEQ ID NO: 21: (Fig. 8,
                                        Aminosäuresequenz)

  (i) SEQUENZ CHARAKTERISTIKA:
       (A) LÄNGE: 57 Aminosäuren
       (B) ART: Aminosäure
       (C) STRANGFORM: Einzel
       (D) TOPOLOGIE: linear

  (ii) ART DES MOLEKÜLS: Polypeptid

  (v) ART DES FRAGMENTS: inneres

  (xi) SEQUENZBESCHREIBUNG: SEQ ID NO: 21:

Gly Val Pro Gly Gly Gly Ala Gly Gly Gly Gly Gly Arg Asp
Val Ser
    1               5                   10
15

Gly Gly Pro Ser Asp Gly Leu Gly Gly Gly Arg Gly Gly Gly
Gly Gly
               20                  25                  30

Gly Asp Ser Gly Gly Met Met Gly Arg Gly Gly Arg Met Leu
Gly Ala
               35                  40                  45

Ser Val Asp Arg Thr Tyr Arg Leu Asn
       50                  55

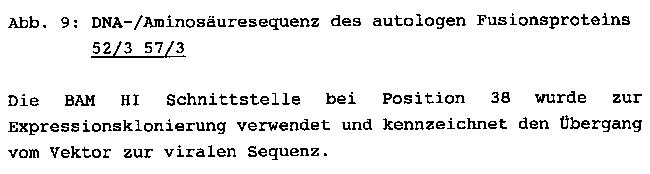

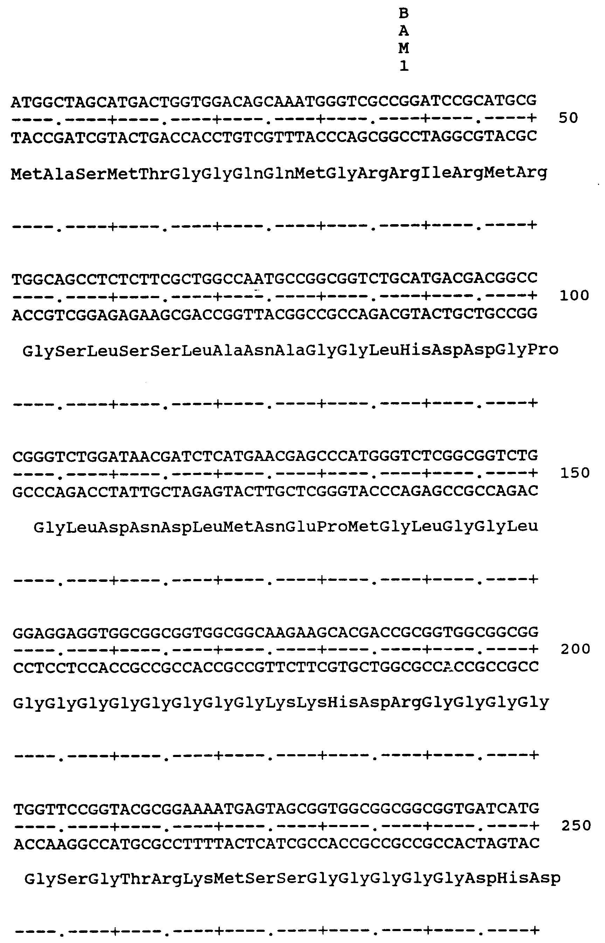

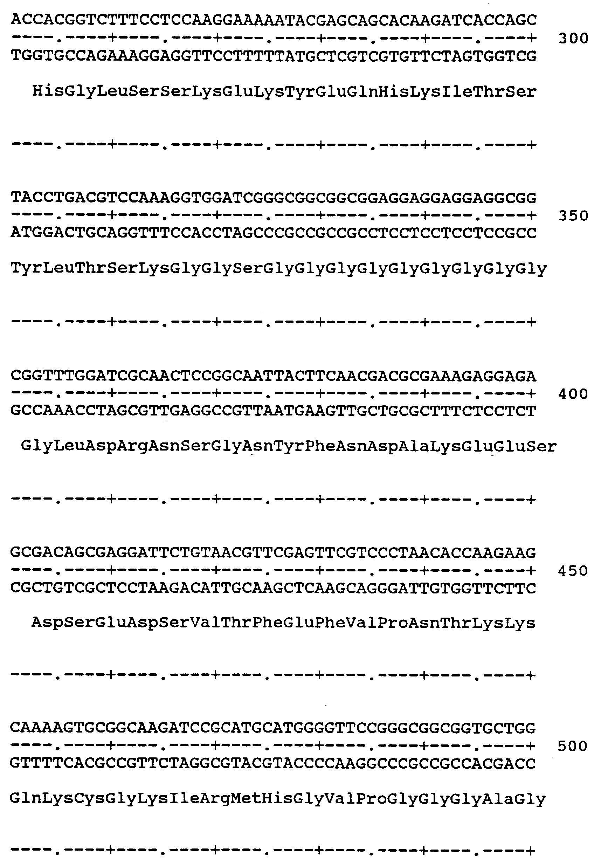

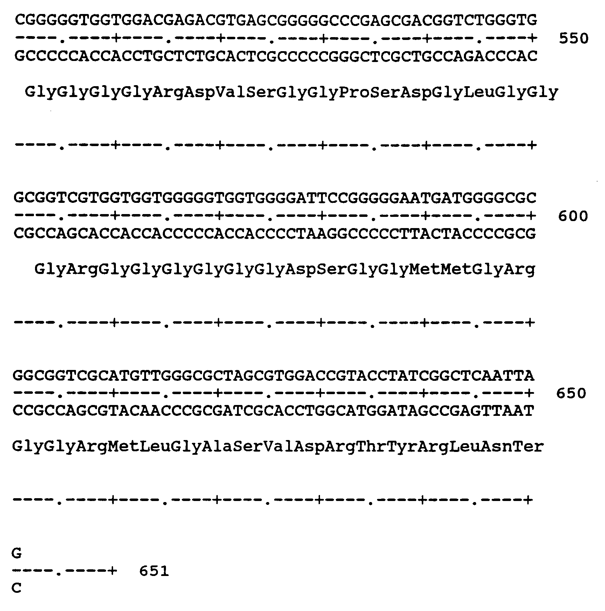

(2) INFORMATION ZU SEQ ID NO: 22: (Fig. 9, oberer Strang)

    (i) SEQUENZ CHARAKTERISTIKA:
       (A) LÄNGE: 651 Basenpaare
       (B) ART: DNA
       (C) STRANGFORM: Einzel
       (D) TOPOLOGIE: linear

    (ii) ART DES MOLEKÜLS: Genom-DNA

    (xi) SEQUENZBESCHREIBUNG: SEQ ID NO: 22:

```
ATGGCTAGCA TGACTGGTGG ACAGCAAATG GGTCGCCGGA TCCGCATGCG
TGGCAGCCTC         60

TCTTCGCTGG CCAATGCCGG CGGTCTGCAT GACGACGGCC CGGGTCTGGA
TAACGATCTC        120

ATGAACGAGC CCATGGGTCT CGGCGGTCTG GGAGGAGGTG GCGGCGGTGG
CGGCAAGAAG        180

CACGACCGCG GTGGCGGCGG TGGTTCCGGT ACGCGGAAAA TGAGTAGCGG
TGGCGGCGGC        240

GGTGATCATG ACCACGGTCT TTCCTCCAAG GAAAAATACG AGCAGCACAA
GATCACCAGC        300

TACCTGACGT CCAAAGGTGG ATCGGGCGGC GGCGGAGGAG GAGGAGGCGG
CGGTTTGGAT        360

CGCAACTCCG GCAATTACTT CAACGACGCG AAAGAGGAGA GCGACAGCGA
GGATTCTGTA        420

ACGTTCGAGT TCGTCCCTAA CACCAAGAAG CAAAAGTGCG GCAAGATCCG
CATGCATGGG        480

GTTCCGGGCG GCGGTGCTGG CGGGGGTGGT GGACGAGACG TGAGCGGGGG
CCCGAGCGAC        540

GGTCTGGGTG GCGGTCGTGG TGGTGGGGGT GGTGGGGATT CCGGGGGAAT
GATGGGGCGC        600

GGCGGTCGCA TGTTGGGCGC TAGCGTGGAC CGTACCTATC GGCTCAATTA G
651
```

(2) INFORMATION ZU SEQ ID NO: 23: (Fig. 9, unterer
                                   Strang)

    (i) SEQUENZ CHARAKTERISTIKA:
        (A) LÄNGE: 651 Basenpaare
        (B) ART: DNA
        (C) STRANGFORM: Einzel
        (D) TOPOLOGIE: linear

   (ii) ART DES MOLEKÜLS: Genom-DNA

   (xi) SEQUENZBESCHREIBUNG: SEQ ID NO: 23:

```
TACCGATCGT ACTGACCACC TGTCGTTTAC CCAGCGGCCT AGGCGTACGC
ACCGTCGGAG       60

AGAAGCGACC GGTTACGGCC GCCAGACGTA CTGCTGCCGG GCCCAGACCT
ATTGCTAGAG      120

TACTTGCTCG GGTACCCAGA GCCGCCAGAC CCTCCTCCAC CGCCGCCACC
GCCGTTCTTC      180

GTGCTGGCGC CACCGCCGCC ACCAAGGCCA TGCGCCTTTT ACTCATCGCC
ACCGCCGCCG      240

CCACTAGTAC TGGTGCCAGA AAGGAGGTTC CTTTTTATGC TCGTCGTGTT
CTAGTGGTCG      300

ATGGACTGCA GGTTTCCACC TAGCCCGCCG CCGCCTCCTC CTCCTCCGCC
GCCAAACCTA      360

GCGTTGAGGC CGTTAATGAA GTTGCTGCGC TTTCTCCTCT CGCTGTCGCT
CCTAAGACAT      420

TGCAAGCTCA AGCAGGGATT GTGGTTCTTC GTTTTCACGC CGTTCTAGGC
GTACGTACCC      480

CAAGGCCCGC CGCCACGACC GCCCCCACCA CCTGCTCTGC ACTCGCCCCC
GGGCTCGCTG      540

CCAGACCCAC CGCCAGCACC ACCACCCCCA CCACCCCTAA GGCCCCCTTA
CTACCCCGCG      600

CCGCCAGCGT ACAACCCGCG ATCGCACCTG GCATGGATAG CCGAGTTAAT C
651
```

(2) INFORMATION ZU SEQ ID NO: 24: (Fig. 9,
Aminosäuresequenz)

    (i) SEQUENZ CHARAKTERISTIKA:
        (A) LÄNGE: 216 Aminosäuren
        (B) ART: Aminosäure
        (C) STRANGFORM: Einzel
        (D) TOPOLOGIE: linear

   (ii) ART DES MOLEKÜLS: Polypeptid

   (v) ART DES FRAGMENTS: inneres

   (xi) SEQUENZBESCHREIBUNG: SEQ ID NO: 24:

```
Met Ala Ser Met Thr Gly Gly Gln Gln Met Gly Arg Arg Ile
Arg Met
  1               5                       10
 15

Arg Gly Ser Leu Ser Ser Leu Ala Asn Ala Gly Gly Leu His
Asp Asp
              20                  25                  30

Gly Pro Gly Leu Asp Asn Asp Leu Met Asn Glu Pro Met Gly
Leu Gly
          35                  40                  45

Gly Leu Gly Gly Gly Gly Gly Gly Gly Gly Lys Lys His Asp
Arg Gly
      50                  55                  60

Gly Gly Gly Gly Ser Gly Thr Arg Lys Met Ser Ser Gly Gly
Gly Gly
      65                  70                  75
 80

Gly Asp His Asp His Gly Leu Ser Ser Lys Glu Lys Tyr Glu
Gln His
                  85                  90
 95

Lys Ile Thr Ser Tyr Leu Thr Ser Lys Gly Gly Ser Gly Gly
Gly Gly
              100                 105                 110

Gly Gly Gly Gly Gly Gly Leu Asp Arg Asn Ser Gly Asn Tyr
Phe Asn
          115                 120                 125

Asp Ala Lys Glu Glu Ser Asp Ser Glu Asp Ser Val Thr Phe
Glu Phe
          130                 135                 140
```

```
        Val Pro Asn Thr Lys Lys Gln Lys Cys Gly Lys Ile Arg Met
    His Gly
        145                 150                 155
    160

        Val Pro Gly Gly Gly Ala Gly Gly Gly Gly Gly Arg Asp Val
    Ser Gly
                        165                 170
    175

        Gly Pro Ser Asp Gly Leu Gly Gly Gly Arg Gly Gly Gly Gly
    Gly Gly
                        180                 185                 190

        Asp Ser Gly Gly Met Met Gly Arg Gly Gly Arg Met Leu Gly
    Ala Ser
                        195                 200                 205

        Val Asp Arg Thr Tyr Arg Leu Asn
        210                 215
```

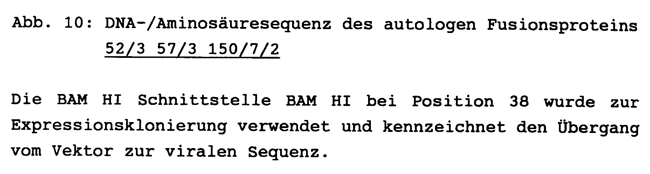

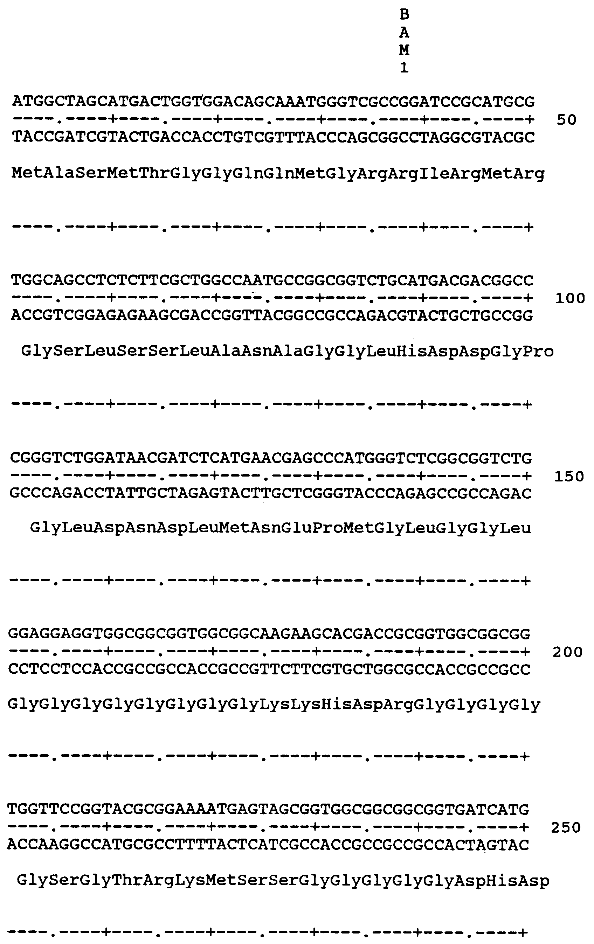

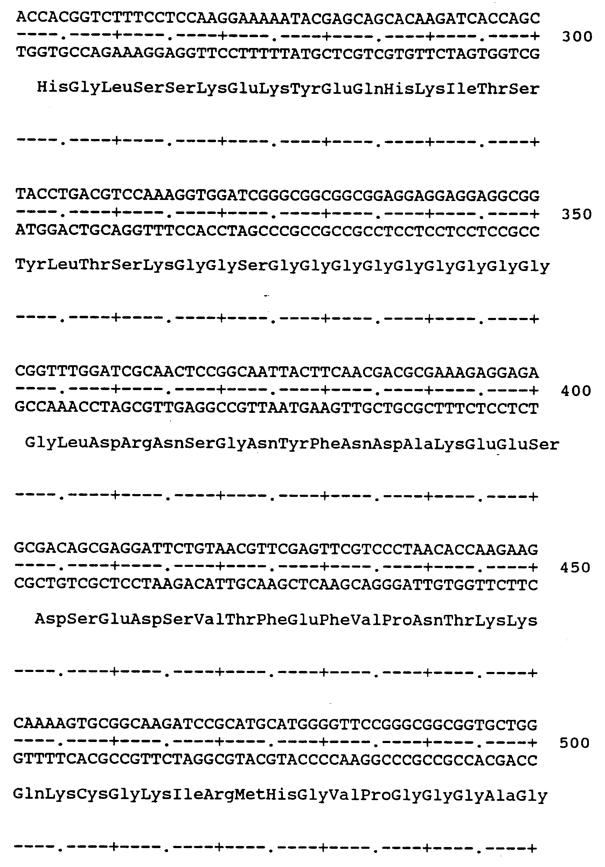

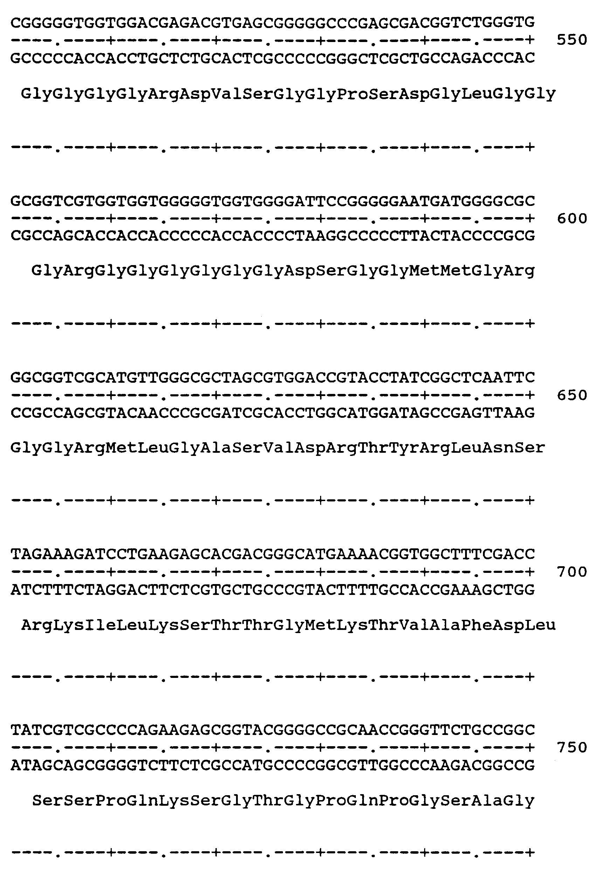

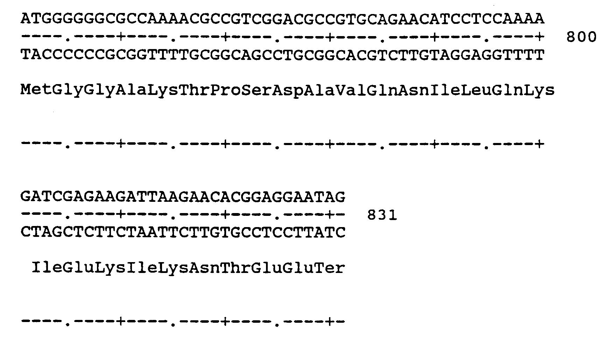

(2) INFORMATION ZU SEQ ID NO: 25: (Fig. 10, oberer
                                        Strang)

     (i) SEQUENZ CHARAKTERISTIKA:
          (A) LÄNGE: 831 Basenpaare
          (B) ART: DNA
          (C) STRANGFORM: Einzel
          (D) TOPOLOGIE: linear

     (ii) ART DES MOLEKÜLS: Genom-DNA

     (xi) SEQUENZBESCHREIBUNG: SEQ ID NO: 25:

     ATGGCTAGCA TGACTGGTGG ACAGCAAATG GGTCGCCGGA TCCGCATGCG
TGGCAGCCTC          60

     TCTTCGCTGG CCAATGCCGG CGGTCTGCAT GACGACGGCC CGGGTCTGGA
TAACGATCTC          120

     ATGAACGAGC CCATGGGTCT CGGCGGTCTG GGAGGAGGTG GCGGCGGTGG
CGGCAAGAAG          180

     CACGACCGCG GTGGCGGCGG TGGTTCCGGT ACGCGGAAAA TGAGTAGCGG
TGGCGGCGGC          240

     GGTGATCATG ACCACGGTCT TTCCTCCAAG GAAAAATACG AGCAGCACAA
GATCACCAGC          300

     TACCTGACGT CCAAAGGTGG ATCGGGCGGC GGCGGAGGAG GAGGAGGCGG
CGGTTTGGAT          360

     CGCAACTCCG GCAATTACTT CAACGACGCG AAAGAGGAGA GCGACAGCGA
GGATTCTGTA          420

     ACGTTCGAGT TCGTCCCTAA CACCAAGAAG CAAAAGTGCG GCAAGATCCG
CATGCATGGG          480

     GTTCCGGGCG GCGGTGCTGG CGGGGGTGGT GGACGAGACG TGAGCGGGGG
CCCGAGCGAC          540

     GGTCTGGGTG GCGGTCGTGG TGGTGGGGGT GGTGGGGATT CCGGGGGAAT
GATGGGGCGC          600

     GGCGGTCGCA TGTTGGGCGC TAGCGTGGAC CGTACCTATC GGCTCAATTC
TAGAAAGATC          660

     CTGAAGAGCA CGACGGGCAT GAAAACGGTG GCTTTCGACC TATCGTCGCC
CCAGAAGAGC          720

     GGTACGGGGC CGCAACCGGG TTCTGCCGGC ATGGGGGGCG CCAAAACGCC
GTCGGACGCC          780

GTGCAGAACA TCCTCCAAAA GATCGAGAAG ATTAAGAACA CGGAGGAATA G

831

(2) INFORMATION ZU SEQ ID NO: 26: (Fig. 10, unterer
Strang)

    (i) SEQUENZ CHARAKTERISTIKA:
        (A) LÄNGE: 831 Basenpaare
        (B) ART: DNA
        (C) STRANGFORM: Einzel
        (D) TOPOLOGIE: linear

   (ii) ART DES MOLEKÜLS: Genom-DNA

   (xi) SEQUENZBESCHREIBUNG: SEQ ID NO: 26:

```
TACCGATCGT ACTGACCACC TGTCGTTTAC CCAGCGGCCT AGGCGTACGC
ACCGTCGGAG          60

AGAAGCGACC GGTTACGGCC GCCAGACGTA CTGCTGCCGG GCCCAGACCT
ATTGCTAGAG         120

TACTTGCTCG GGTACCCAGA GCCGCCAGAC CCTCCTCCAC CGCCGCCACC
GCCGTTCTTC         180

GTGCTGGCGC CACCGCCGCC ACCAAGGCCA TGCGCCTTTT ACTCATCGCC
ACCGCCGCCG         240

CCACTAGTAC TGGTGCCAGA AAGGAGGTTC CTTTTTATGC TCGTCGTGTT
CTAGTGGTCG         300

ATGGACTGCA GGTTTCCACC TAGCCCGCCG CCGCCTCCTC CTCCTCCGCC
GCCAAACCTA         360

GCGTTGAGGC CGTTAATGAA GTTGCTGCGC TTTCTCCTCT CGCTGTCGCT
CCTAAGACAT         420

TGCAAGCTCA AGCAGGGATT GTGGTTCTTC GTTTTCACGC CGTTCTAGGC
GTACGTACCC         480

CAAGGCCCGC CGCCACGACC GCCCCCACCA CCTGCTCTGC ACTCGCCCCC
GGGCTCGCTG         540

CCAGACCCAC CGCCAGCACC ACCACCCCCA CCACCCCTAA GGCCCCCTTA
CTACCCCGCG         600

CCGCCAGCGT ACAACCCGCG ATCGCACCTG GCATGGATAG CCGAGTTAAG
ATCTTTCTAG         660

GACTTCTCGT GCTGCCCGTA CTTTTGCCAC CGAAAGCTGG ATAGCAGCGG
GGTCTTCTCG         720

CCATGCCCCG GCGTTGGCCC AAGACGGCCG TACCCCCCGC GGTTTTGCGG
CAGCCTGCGG         780

CACGTCTTGT AGGAGGTTTT CTAGCTCTTC TAATTCTTGT GCCTCCTTAT C
831
```

(2) INFORMATION ZU SEQ ID NO: 27: (Fig. 10,
Aminosäuresequenz)

    (i) SEQUENZ CHARAKTERISTIKA:
        (A) LÄNGE: 276 Aminosäuren
        (B) ART: Aminosäure
        (C) STRANGFORM: Einzel
        (D) TOPOLOGIE: linear

   (ii) ART DES MOLEKÜLS: Polypeptid

   (v) ART DES FRAGMENTS: inneres

  (xi) SEQUENZBESCHREIBUNG: SEQ ID NO: 27:

```
Met Ala Ser Met Thr Gly Gly Gln Gln Met Gly Arg Arg Ile
Arg Met
  1             5                   10
 15

Arg Gly Ser Leu Ser Ser Leu Ala Asn Ala Gly Gly Leu His
Asp Asp
             20                  25                   30

Gly Pro Gly Leu Asp Asn Asp Leu Met Asn Glu Pro Met Gly
Leu Gly
             35                  40                   45

Gly Leu Gly Gly Gly Gly Gly Gly Gly Gly Lys Lys His Asp
Arg Gly
      50                  55                   60

Gly Gly Gly Gly Ser Gly Thr Arg Lys Met Ser Ser Gly Gly
Gly Gly
      65                  70                   75
 80

Gly Asp His Asp His Gly Leu Ser Ser Lys Glu Lys Tyr Glu
Gln His
                 85                  90
 95

Lys Ile Thr Ser Tyr Leu Thr Ser Lys Gly Gly Ser Gly Gly
Gly Gly
             100                 105                  110

Gly Gly Gly Gly Gly Gly Leu Asp Arg Asn Ser Gly Asn Tyr
Phe Asn
             115                 120                  125

Asp Ala Lys Glu Glu Ser Asp Ser Glu Asp Ser Val Thr Phe
Glu Phe
             130                 135                  140
```

```
        Val Pro Asn Thr Lys Lys Gln Lys Cys Gly Lys Ile Arg Met
    His Gly
        145             150             155
    160

        Val Pro Gly Gly Gly Ala Gly Gly Gly Gly Gly Arg Asp Val
    Ser Gly
                        165             170
    175

        Gly Pro Ser Asp Gly Leu Gly Gly Gly Arg Gly Gly Gly Gly
    Gly Gly
                        180             185             190

        Asp Ser Gly Gly Met Met Gly Arg Gly Gly Arg Met Leu Gly
    Ala Ser
                        195             200             205

        Val Asp Arg Thr Tyr Arg Leu Asn Ser Arg Lys Ile Leu Lys
    Ser Thr
        210             215             220

        Thr Gly Met Lys Thr Val Ala Phe Asp Leu Ser Ser Pro Gln
    Lys Ser
        225             230             235
    240

        Gly Thr Gly Pro Gln Pro Gly Ser Ala Gly Met Gly Gly Ala
    Lys Thr
                        245             250
    255

        Pro Ser Asp Ala Val Gln Asn Ile Leu Gln Lys Ile Glu Lys
    Ile Lys
                        260             265             270

    Asn Thr Glu Glu
            275
```

## Patentansprüche

1. Polypeptid umfassend wenigstens eine vom Cytomegalievirus (HCMV) stammende Aminosäuresequenz, dadurch gekennzeichnet, daß diese Sequenz ausgewählt ist aus:

a) dem Leserahmen UL 57 des HCM-Virus, wobei dieser Leserahmen für das Major-DNA-binding protein codiert und eine Homologie von wenigstens 60 % zu der Aminosäuresequenz

```
Gly Val Pro Gly Gly Gly Ala Gly Gly Gly Gly Gly Arg Asp Val
Ser Gly Gly Pro Ser Asp Gly Leu Gly Gly Gly Arg Gly Gly Gly
Gly Gly Gly Asp Ser Gly Gly Met Met Gly Arg Gly Gly Arg Met
Leu Gly Ala Ser Val Asp Arg Thr Tyr Arg Leu Asn
```

aufweist und/oder

b) dem C-terminalen Bereich des Tegumentproteins pp150 des Cytomegalievirus und wenigstens einem weiteren Fragment aus einem anderen antigenen Protein, das von dem humanen Cytomegalievirus stammt, wobei das Polypeptid gemäß b) als Fusionsprotein vorliegt.

2. Polypeptid nach Anspruch 1, dadurch gekennzeichnet, daß das Polypeptid gemäß Alternative a) eine Homologie von wenigstens 80 % zu der in Anspruch 1 dargestellten Aminosäuresequenz hat.

3. Polypeptid nach Anspruch 1, dadurch gekennzeichnet, daß das Polypeptid der Aminosäuresequenz nach Anspruch 1, Alternative a), oder einer Teilsequenz davon entspricht.

4. Polypeptid nach einem der Ansprüche 1 bis 3, dadurch gekennzeichnet, daß es eine Länge von wenigstens 10 Aminosäuren hat.

5. Polypeptid nach einem der vorhergehenden Ansprüche, dadurch gekennzeichnet, daß es durch chemische Methoden synthetisiert wurde.

6. Fusionsprotein, dadurch gekennzeichnet, daß es eine Aminosäuresequenz eines Polypeptids nach einem der Ansprüche 1 bis 4 umfaßt.

7. Fusionsprotein nach Anspruch 1, Alternative b), dadurch gekennzeichnet, daß das andere antigene, von dem humanen Cytomegalievirus stammende Protein das p52-Protein ist.

8. Fusionsprotein nach Anspruch 7, dadurch gekennzeichnet, daß das andere antigene, von dem humanen Cytomegalievirus stammende Protein aus dem C-terminalen Bereich des p52-Proteins stammt.

9. Fusionsprotein nach einem der Ansprüche 7 oder 8, dadurch gekennzeichnet, daß das Fusionsprotein eine Aminosäuresequenz aufweist, die eine Homologie von wenigstens 80 % zu der in Abbildung 2 gezeigten Aminosäuresequenz aufweist.

10. Fusionsprotein nach Anspruch 9, dadurch gekennzeichnet, daß das Fusionsprotein die in Abbildung 2 gezeigte Aminosäuresequenz aufweist.

11. Fusionsprotein nach einem der Ansprüche 6 bis 10, dadurch gekennzeichnet, daß das Fusionsprotein nicht mehr als 25 Aminosäuren aufweist, die von einem bakteriellen Protein oder dem Vektor, mit dessen Hilfe das Fusionsprotein exprimiert wird, herstammen.

12. Fusionsprotein nach Anspruch 6, dadurch gekennzeichnet, daß ein Teil des Fusionsproteins von der Glutathion-S-Transferase herstammt.

13. Fusionsprotein nach einem der Ansprüche 6 bis 12, dadurch gekennzeichnet, daß ein Teil des Fusionsproteins von dem Polypeptid gemäß Variante a) des Anspruchs 1 und der andere Teil von wenigstens einem anderen Protein, insbesondere einem anderen antigenen Protein, des humanen Cytomegalievirus herstammt.

14. Fusionsprotein nach Anspruch 13, dadurch gekennzeichnet, daß es sich bei dem anderen antigenen Protein des humanen Cytomegalievirus um das pp150-Tegumentprotein handelt.

15. Fusionsprotein nach Anspruch 13 oder 14, dadurch gekennzeichnet, daß es weiterhin einen Teil der Aminosäuresequenz des p52-Proteins des humanen Cytomegalievirus aufweist.

16. Testkit zum Nachweis von Antikörpern, insbesondere IgM-Antikörpern gegen das Cytomegalievirus, dadurch gekennzeichnet, daß es wenigstens ein Polypeptid nach einem der Ansprüche 1 bis 5 und/oder wenigstens ein Fusionsprotein nach einem der Ansprüche 6 bis 15 umfaßt.

17. Testkit nach Anspruch 16, dadurch gekennzeichnet, daß es sich um ein Testkit zur Durchführung eines ELISA-Tests handelt.

18. Verfahren zum Nachweis von Antikörpern, insbesondere IgM-Antikörpern gegen das Cytomegalievirus, dadurch gekennzeichnet, daß man eine zu untersuchende Probe mit einem Polypeptid nach einem der Ansprüche 1 bis 5 und/oder einem Fusionsprotein nach einem der Ansprüche 6 bis 15 zusammenbringt und den Antikörper-Polypeptid-Komplex und/oder den Antikörper-Fusionsprotein-Komplex mit einer Nachweiskomponente detektiert.

19. Verfahren nach Anspruch 18, dadurch gekennzeichnet, daß die Nachweiskomponente ein Antihumanantikörper ist, der gekoppelt ist mit einem Indikatormolekül, insbesondere Meerrettich Peroxidase.

20. Oligonucleotid, dadurch gekennzeichnet, daß es eine Sequenz von Nucleotiden enthält, die für eine Aminosäuresequenz codiert, die zumindest einem Teil eines Polypeptids gemäß Anspruch 1 entspricht.

21. Oligonucleotid nach Anspruch 20, dadurch gekennzeichnet, daß es 12-30 Basen lang ist.

22. Verwendung eines Oligonucleotids gemäß Anspruch 20 oder 21 zum Nachweis einer Infektion mit einem Cytomegalievirus mit Hilfe eines Verfahrens zur Vervielfältigung von Nucleinsäuren.

PCC15012.SEQ

```
    ----,----+----,----+----,----+----,----+----,----+
  1 GAATTCTATTCCTCCGTGTTCTTAATC                          27
```

PCC15013.SEQ

```
    ----,----+----,----+----,----+----,----+----,----+
  1 CGGATCCTGAAGAGCACGACGGGCAT                           26
```

PCC525.SEQ

```
    ----,----+----,----+----,----+----,----+----,----+
  1 GGATCCGCATGCGTGGCAGCCTCTCTTCGCTGGCC                  35
```

PCC526.SEQ

```
    ----,----+----,----+----,----+----,----+----,----+
  1 GAATTCAGATCTTGCCGCACTTTTGCTTCT                       30
```

**Abb. 1:** Auflistung der zur Klonierung des autologen Fusionsproteins 52/3 | 105/7/2 verwendeten PCR-Primer.

```
B
A
M
1

GGATCCGCATGCGTGGCAGCCTCTCTTCGCTGGCCAATGCCGGCGGTCTG
----.----+----.----+----.----+----.----+----.----+    50
CCTAGGCGTACGCACCGTCGGAGAGAAGCGACCGGTTACGGCCGCCAGAC


  IleArgMetArgGlySerLeuSerSerLeuAlaAsnAlaGlyGlyLeu
----.----+----.----+----.----+----.----+----.----+


CATGACGACGGCCCGGGTCTGGATAACGATCTCATGAACGAGCCCATGGG
----.----+----.----+----.----+----.----+----.----+    100
GTACTGCTGCCGGGCCCAGACCTATTGCTAGAGTACTTGCTCGGGTACCC


HisAspAspGlyProGlyLeuAspAsnAspLeuMetAsnGluProMetGly
----.----+----.----+----.----+----.----+----.----+


TCTCGGCGGTCTGGGAGGAGGTGGCGGCGGTGGCGGCAAGAAGCACGACC
----.----+----.----+----.----+----.----+----.----+    150
AGAGCCGCCAGACCCTCCTCCACCGCCGCCACCGCCGTTCTTCGTGCTGG


  LeuGlyGlyLeuGlyGlyGlyGlyGlyGlyGlyGlyLysLysHisAspArg
----.----+----.----+----.----+----.----+----.----+


GCGGTGGCGGCGGTGGTTCCGGTACGCGGAAAATGAGTAGCGGTGGCGGC
----.----+----.----+----.----+----.----+----.----+    200
CGCCACCGCCGCCACCAAGGCCATGCGCCTTTTACTCATCGCCACCGCCG


  GlyGlyGlyGlyGlySerGlyThrArgLysMetSerSerGlyGlyGly
----.----+----.----+----.----+----.----+----.----+


GGCGGTGATCATGACCACGGTCTTTCCTCCAAGGAAAAATACGAGCAGCA
----.----+----.----+----.----+----.----+----.----+    250
CCGCCACTAGTACTGGTGCCAGAAAGGAGGTTCCTTTTTATGCTCGTCGT


  GlyGlyAspHisAspHisGlyLeuSerSerLysGluLysTyrGluGlnHis
----.----+----.----+----.----+----.----+----.----+
```

**Abb. 2:** Sequenz und resultierende AS-Sequenz des für das autologe Fusionsprotein codierenden DNA-Fragments. Die zur weiteren Klonierung verwendeten Restriktionsschnittstellen Bam HI und Eco RI sind gekennzeichnet.

```
CAAGATCACCAGCTACCTGACGTCCAAAGGTGGATCGGGCGGCGGCGGAG
----.----+----.----+----.----+----.----+----.----+   300
GTTCTAGTGGTCGATGGACTGCAGGTTTCCACCTAGCCCGCCGCCGCCTC


  LysIleThrSerTyrLeuThrSerLysGlyGlySerGlyGlyGlyGlyGly
----.----+----.----+----.----+----.----+----.----+


GAGGAGGAGGCGGCGGTTTGGATCGCAACTCCGGCAATTACTTCAACGAC
----.----+----.----+----.----+----.----+----.----+   350
CTCCTCCTCCGCCGCCAAACCTAGCGTTGAGGCCGTTAATGAAGTTGCTG


   GlyGlyGlyGlyGlyLeuAspArgAsnSerGlyAsnTyrPheAsnAsp
----.----+----.----+----.----+----.----+----.----+


GCGAAAGAGGAGAGCGACAGCGAGGATTCTGTAACGTTCGAGTTCGTCCC
----.----+----.----+----.----+----.----+----.----+   400
CGCTTTCTCCTCTCGCTGTCGCTCCTAAGACATTGCAAGCTCAAGCAGGG


AlaLysGluGluSerAspSerGluAspSerValThrPheGluPheValPro
----.----+----.----+----.----+----.----+----.----+


TAACACCAAGAAGCAAAAGTGCGGCAAGATCCTGAAGAGCACGACGGGCA
----.----+----.----+----.----+----.----+----.----+   450
ATTGTGGTTCTTCGTTTTCACGCCGTTCTAGGACTTCTCGTGCTGCCCGT


  AsnThrLysLysGlnLysCysGlyLysIleLeuLysSerThrThrGlyMet
----.----+----.----+----.----+----.----+----.----+


TGAAAACGGTGGCTTTCGACCTATCGTCGCCCCAGAAGAGCGGTACGGGG
----.----+----.----+----.----+----.----+----.----+   500
ACTTTTGCCACCGAAAGCTGGATAGCAGCGGGGTCTTCTCGCCATGCCCC


  LysThrValAlaPheAspLeuSerSerProGlnLysSerGlyThrGly
----.----+----.----+----.----+----.----+----.----+
```

MAPSEQ V5.33 52315072.SEQ(1,607)

```
        CCGCAACCGGGTTCTGCCGGCATGGGGGGCGCCAAAACGCCGTCGGACGC
        ----.----+----.----+----.----+----.----+----.----+   550
        GGCGTTGGCCCAAGACGGCCGTACCCCCCGCGGTTTTGCGGCAGCCTGCG


        ProGlnProGlySerAlaGlyMetGlyGlyAlaLysThrProSerAspAla
        ----.----+----.----+----.----+----.----+----.----+


        CGTGCAGAACATCCTCCAAAAGATCGAGAAGATTAAGAACACGGAGGAAT
        ----.----+----.----+----.----+----.----+----.----+   600
        GCACGTCTTGTAGGAGGTTTTCTAGCTCTTCTAATTCTTGTGCCTCCTTA


        ValGlnAsnIleLeuGlnLysIleGluLysIleLysAsnThrGluGluTer
        ----.----+----.----+----.----+----.----+----.----+


        E
        C
        R
        1

        AGAATTC
        ----.----+   607
        TCTTAAG
```

**Abb. 3:** Analytik der verschiedenen Reinigungsstufen des rekombinanten 52/3 150/7/2 mit SDS-PAG-Elektrophorese

1: Vor Induktion
_. 3 h nach Induktion
3: lösliches Protein
4: Nach Lyse mit Lysozym und Ultraschall
5: nach fraktionierter Ammoniumsulfatfällung
6: Nach Kationenaustauschchromatographie
7: nach Konzentrierung durch Ultrafiltration
8: MW-Marker

Abb. 4: Reaktivität des gereinigten autologen Fusionsproteins 52/3 | 150/7/2 im IgM-ELISA mit Verlaufsseren von Transplantationspatienten mit akuter HCMV-Primärinfektion.

**Tab. 1:** IgM-Reaktivität der rekombinanten Antigene mit Seren gesunder Blutspender

| A. CMV - seropositiv (n = 54) | positiv gesamt (OD > 0,3) | OD < 0,5 | OD 0,5 - 1,0 | OD > 1,0 |
|---|---|---|---|---|
| 150/7 | 19 | 7 | 8 | 4 |
| 52/3 | 1 | — | 1 | — |
| 52/3 \| 105/7/2 | 1 | — | 1 | — |

| B. CMV - seronegativ (n = 54) | positiv gesamt (OD > 0,3) | OD < 0,5 | OD 0,5 - 1,0 | OD > 1,0 |
|---|---|---|---|---|
| 150/7 | — | — | — | — |
| 52/3 | — | — | — | — |
| 52/3 \| 150/7/2 | — | — | — | — |

Abb. 5

EP 0 702 083 A2

**Tab. 2:** OD-Werte im IgM-ELISA mit Seren immunkompetenter Patienten mit akuter HCMV-Infektion. Die schraffierten Werte liegen über dem cut-off von 0,3.

| Serum | 150/7 | 52/3 | 52/3 \| 150/7/2 |
|---|---|---|---|
| 75332 | 1,576 | 0,353 | 0,736 |
| 81297 | 3,0 | 0,533 | 1,494 |
| 83381 | 3,0 | 0,971 | 2,654 |
| 103850 | 2,473 | 2,305 | 2,189 |
| 112690 | 0,411 | 0,077 | 0,167 |
| 132847 | 1,268 | 0,249 | 0,111 |
| 138076 | 3,0 | 2,019 | 2,518 |
| 145608 | 0,289 | 1,863 | 2,493 |
| 65327 | 1,448 | 0,282 | 1,140 |
| 0162 | 2,742 | 0,203 | 1,434 |
| 1012 | 2,736 | 0,139 | 1,506 |
| 9344 | 2,689 | 0,065 | 2,180 |
| 1744 | 2,427 | 0,030 | 1,704 |
| 1150 | 3,0 | 0,258 | 1,255 |
| 3313 | 3,0 | 0,165 | 0,542 |

Abb. 6

Abb. 7: Auflistung der zur Klonierung der verschiedenen
Fragmente des Leserahmens UL 57 verwendeten PCR-
Primer bzw. Oligonucleotide

PCCUL571.SEQ

```
    ----,----+----,----+----,----+----,----+----,----+
  1 GGATCCGCATGCATCACGACCGCCTGCTGGACT                     33
```

     PCCUL571.SEQ

```
    ----,----+----,----+----,----+----,----+----,----+
  1 GAATTCTTAGTTGTTGATACCCGCATATT                         29
```

     PCCUL572

PCCUL577

```
    ----,----+----,----+----,----+----,----+----,----+
  1 GGATCCGCATGCATGGGGTTCCGGGCGGCGGTGC                    34
```

     PCCUL577.SEQ

```
    ----,----+----,----+----,----+----,----+----,----+
  1 GAATTCTCTAGAATTGAGCCGATAGGTACGG                       31
```

     PCCUL578.SEQ

HC572SY1.SEQ

```
    ----,----+----,----+----,----+----,----+----,----+
  1 GATCCCCTCTAGAGACGCTCAGCGTCTTACTGACGCTGCAGG            42
```

     HC572SY1.SEQ

HC572SY2.SEQ

```
    ----,----+----,----+----,----+----,----+----,----+
  1 AATCTCTGCAGCGTCAGTAAGACGCTGAGCGTCTCTAGAGGG            42
```

     HC572SY2.SEQ

HC572SY3.SEQ

```
    ----,----+----,----+----,----+----,----+----,----+
  1 GGTGGTGAAGTTCATGACCTTTCTGCTCTTTTCGCTCCGTCTGGTGTTGG    50
 51 TGCAGCTTCTGGTGTTGGTGGG                                72
```

     HC572SY3.SEQ

HC572SY4.SEQ

```
     ----,----+----,----+----,----+----,----+----,----+
   1 AATCTCCACCAACACCAGAAGCTGCACCAACACCAGACGGAGCGAAAAGA 50
  51 GCAGAAAGGTCATGAACTTCACCACCTGCA                      80
```

HC572SY4.SEQ


HC572SY5.SEQ

```
     ----,----+----,----+----,----+----,----+----,----+
   1 TGGTGGTCTGCTTCTTGGTGAATCTGTTGCTGGTAACTCTATCTGCTTCG 50
  51 GTGTCCCGGGG                                         61
```

HC572SY5.SEQ


HC572SY6.SEQ

```
     ----,----+----,----+----,----+----,----+----,----+
   1 AATTCCCCGGGACACCGAAGCAGATAGAGTTACCAGCAACAGATTCACCA 50
  51 AGAAGCAGACCACCACC                                   67
```

```
GGGGTTCCGGGCGGCGGTGCTGGCGGGGGTGGTGGACGAGACGTGAGCGG
----.----+----.----+----.----+----.----+----.----+   50
CCCCAAGGCCCGCCGCCACGACCGCCCCCACCACCTGCTCTGCACTCGCC

GlyValProGlyGlyGlyAlaGlyGlyGlyGlyGlyArgAspValSerGly


----.----+----.----+----.----+----.----+----.----+


GGGCCCGAGCGACGGTCTGGGTGGCGGTCGTGGTGGTGGGGGTGGTGGGG
----.----+----.----+----.----+----.----+----.----+   100
CCCGGGCTCGCTGCCAGACCCACCGCCAGCACCACCACCCCCACCACCCC

 GlyProSerAspGlyLeuGlyGlyGlyArgGlyGlyGlyGlyGlyGlyAsp


----.----+----.----+----.----+----.----+----.----+


ATTCCGGGGGAATGATGGGGCGCGGCGGTCGCATGTTGGGCGCTAGCGTG
----.----+----.----+----.----+----.----+----.----+   150
TAAGGCCCCCTTACTACCCCGCGCCGCCAGCGTACAACCCGCGATCGCAC

 SerGlyGlyMetMetGlyArgGlyGlyArgMetLeuGlyAlaSerVal


----.----+----.----+----.----+----.----+----.----+


GACCGTACCTATCGGCTCAAT
----.----+----.----+-   171
CTGGCATGGATAGCCGAGTTA

AspArgThrTyrArgLeuAsn


----.----+----.----+-
```

Abb. 8 :   DNA-Sequenz und resultierende Aminosäuresequenz des
           immundominanten Fragments UL57/3

Abb. 9: DNA-/Aminosäuresequenz des autologen Fusionsproteins
52/3 57/3

Die BAM HI Schnittstelle bei Position 38 wurde zur
Expressionsklonierung verwendet und kennzeichnet den Übergang
vom Vektor zur viralen Sequenz.

```
                                        B
                                        A
                                        M
                                        1

ATGGCTAGCATGACTGGTGGACAGCAAATGGGTCGCCGGATCCGCATGCG
----.----+----.----+----.----+----.----+----.----+   50
TACCGATCGTACTGACCACCTGTCGTTTACCCAGCGGCCTAGGCGTACGC

MetAlaSerMetThrGlyGlyGlnGlnMetGlyArgArgIleArgMetArg


----.----+----.----+----.----+----.----+----.----+


TGGCAGCCTCTCTTCGCTGGCCAATGCCGGCGGTCTGCATGACGACGGCC
----.----+----.----+----.----+----.----+----.----+   100
ACCGTCGGAGAGAAGCGACCGGTTACGGCCGCCAGACGTACTGCTGCCGG

 GlySerLeuSerSerLeuAlaAsnAlaGlyGlyLeuHisAspAspGlyPro


----.----+----.----+----.----+----.----+----.----+


CGGGTCTGGATAACGATCTCATGAACGAGCCCATGGGTCTCGGCGGTCTG
----.----+----.----+----.----+----.----+----.----+   150
GCCCAGACCTATTGCTAGAGTACTTGCTCGGGTACCCAGAGCCGCCAGAC

  GlyLeuAspAsnAspLeuMetAsnGluProMetGlyLeuGlyGlyLeu


----.----+----.----+----.----+----.----+----.----+


GGAGGAGGTGGCGGCGGTGGCGGCAAGAAGCACGACCGCGGTGGCGGCGG
----.----+----.----+----.----+----.----+----.----+   200
CCTCCTCCACCGCCGCCACCGCCGTTCTTCGTGCTGGCGCCACCGCCGCC

GlyGlyGlyGlyGlyGlyGlyGlyLysLysHisAspArgGlyGlyGlyGly


----.----+----.----+----.----+----.----+----.----+


TGGTTCCGGTACGCGGAAAAATGAGTAGCGGTGGCGGCGGCGGTGATCATG
----.----+----.----+----.----+----.----+----.----+   250
ACCAAGGCCATGCGCCTTTTACTCATCGCCACCGCCGCCGCCACTAGTAC

  GlySerGlyThrArgLysMetSerSerGlyGlyGlyGlyGlyAspHisAsp


----.----+----.----+----.----+----.----+----.----+
```

```
ACCACGGTCTTTCCTCCAAGGAAAAATACGAGCAGCACAAGATCACCAGC
----.----+----.----+----.----+----.----+----.----+    300
TGGTGCCAGAAAGGAGGTTCCTTTTTATGCTCGTCGTGTTCTAGTGGTCG

  HisGlyLeuSerSerLysGluLysTyrGluGlnHisLysIleThrSer


----.----+----.----+----.----+----.----+----.----+


TACCTGACGTCCAAAGGTGGATCGGGCGGCGGCGGAGGAGGAGGAGGCGG
----.----+----.----+----.----+----.----+----.----+    350
ATGGACTGCAGGTTTCCACCTAGCCCGCCGCCGCCTCCTCCTCCTCCGCC

TyrLeuThrSerLysGlyGlySerGlyGlyGlyGlyGlyGlyGlyGlyGly


----.----+----.----+----.----+----.----+----.----+


CGGTTTGGATCGCAACTCCGGCAATTACTTCAACGACGCGAAAGAGGAGA
----.----+----.----+----.----+----.----+----.----+    400
GCCAAACCTAGCGTTGAGGCCGTTAATGAAGTTGCTGCGCTTTCTCCTCT

  GlyLeuAspArgAsnSerGlyAsnTyrPheAsnAspAlaLysGluGluSer


----.----+----.----+----.----+----.----+----.----+


GCGACAGCGAGGATTCTGTAACGTTCGAGTTCGTCCCTAACACCAAGAAG
----.----+----.----+----.----+----.----+----.----+    450
CGCTGTCGCTCCTAAGACATTGCAAGCTCAAGCAGGGATTGTGGTTCTTC

  AspSerGluAspSerValThrPheGluPheValProAsnThrLysLys


----.----+----.----+----.----+----.----+----.----+


CAAAAGTGCGGCAAGATCCGCATGCATGGGGTTCCGGGCGGCGGTGCTGG
----.----+----.----+----.----+----.----+----.----+    500
GTTTTCACGCCGTTCTAGGCGTACGTACCCCAAGGCCCGCCGCCACGACC

GlnLysCysGlyLysIleArgMetHisGlyValProGlyGlyGlyAlaGly


----.----+----.----+----.----+----.----+----.----+
```

```
CGGGGGTGGTGGACGAGACGTGAGCGGGGGCCCGAGCGACGGTCTGGGTG
----.----+----.----+----.----+----.----+----.----+    550
GCCCCCACCACCTGCTCTGCACTCGCCCCCGGGCTCGCTGCCAGACCCAC

  GlyGlyGlyGlyArgAspValSerGlyGlyProSerAspGlyLeuGlyGly


----.----+----.----+----.----+----.----+----.----+


GCGGTCGTGGTGGTGGGGGGTGGTGGGGGATTCCGGGGGAATGATGGGGCGC
----.----+----.----+----.----+----.----+----.----+    600
CGCCAGCACCACCACCCCCACCACCCCTAAGGCCCCCTTACTACCCCGCG

  GlyArgGlyGlyGlyGlyGlyGlyAspSerGlyGlyMetMetGlyArg


----.----+----.----+----.----+----.----+----.----+


GGCGGTCGCATGTTGGGCGCTAGCGTGGACCGTACCTATCGGCTCAATTA
----.----+----.----+----.----+----.----+----.----+    650
CCGCCAGCGTACAACCCGCGATCGCACCTGGCATGGATAGCCGAGTTAAT

GlyGlyArgMetLeuGlyAlaSerValAspArgThrTyrArgLeuAsnTer


----.----+----.----+----.----+----.----+----.----+


G
----.----+    651
C
```

58

Abb. 10: DNA-/Aminosäuresequenz des autologen Fusionsproteins 52/3 57/3 150/7/2

Die BAM HI Schnittstelle BAM HI bei Position 38 wurde zur Expressionsklonierung verwendet und kennzeichnet den Übergang vom Vektor zur viralen Sequenz.

```
                                         B
                                         A
                                         M
                                         1

ATGGCTAGCATGACTGGTGGACAGCAAATGGGTCGCCGGATCCGCATGCG
----.----+----.----+----.----+----.----+----.----+   50
TACCGATCGTACTGACCACCTGTCGTTTACCCAGCGGCCTAGGCGTACGC

MetAlaSerMetThrGlyGlyGlnGlnMetGlyArgArgIleArgMetArg


----.----+----.----+----.----+----.----+----.----+


TGGCAGCCTCTCTTCGCTGGCCAATGCCGGCGGTCTGCATGACGACGGCC
----.----+----.----+----.----+----.----+----.----+  100
ACCGTCGGAGAGAAGCGACCGGTTACGGCCGCCAGACGTACTGCTGCCGG

 GlySerLeuSerSerLeuAlaAsnAlaGlyGlyLeuHisAspAspGlyPro


----.----+----.----+----.----+----.----+----.----+


CGGGTCTGGATAACGATCTCATGAACGAGCCCATGGGTCTCGGCGGTCTG
----.----+----.----+----.----+----.----+----.----+  150
GCCCAGACCTATTGCTAGAGTACTTGCTCGGGTACCCAGAGCCGCCAGAC

 GlyLeuAspAsnAspLeuMetAsnGluProMetGlyLeuGlyGlyLeu


----.----+----.----+----.----+----.----+----.----+


GGAGGAGGTGGCGGCGGTGGCGGCAAGAAGCACGACCGCGGTGGCGGCGG
----.----+----.----+----.----+----.----+----.----+  200
CCTCCTCCACCGCCGCCACCGCCGTTCTTCGTGCTGGCGCCACCGCCGCC

GlyGlyGlyGlyGlyGlyGlyGlyLysLysHisAspArgGlyGlyGlyGly


----.----+----.----+----.----+----.----+----.----+


TGGTTCCGGTACGCGGAAAATGAGTAGCGGTGGCGGCGGCGGTGATCATG
----.----+----.----+----.----+----.----+----.----+  250
ACCAAGGCCATGCGCCTTTTACTCATCGCCACCGCCGCCGCCACTAGTAC

 GlySerGlyThrArgLysMetSerSerGlyGlyGlyGlyGlyAspHisAsp


----.----+----.----+----.----+----.----+----.----+
```

```
ACCACGGTCTTTCCTCCAAGGAAAAATACGAGCAGCACAAGATCACCAGC
----.----+----.----+----.----+----.----+----.----+    300
TGGTGCCAGAAAGGAGGTTCCTTTTTATGCTCGTCGTGTTCTAGTGGTCG

   HisGlyLeuSerSerLysGluLysTyrGluGlnHisLysIleThrSer


----.----+----.----+----.----+----.----+----.----+


TACCTGACGTCCAAAGGTGGATCGGGCGGCGGCGGAGGAGGAGGAGGCGG
----.----+----.----+----.----+----.----+----.----+    350
ATGGACTGCAGGTTTCCACCTAGCCCGCCGCCGCCTCCTCCTCCTCCGCC

TyrLeuThrSerLysGlyGlySerGlyGlyGlyGlyGlyGlyGlyGlyGly


----.----+----.----+----.----+----.----+----.----+


CGGTTTGGATCGCAACTCCGGCAATTACTTCAACGACGCGAAAGAGGAGA
----.----+----.----+----.----+----.----+----.----+    400
GCCAAACCTAGCGTTGAGGCCGTTAATGAAGTTGCTGCGCTTTCTCCTCT

  GlyLeuAspArgAsnSerGlyAsnTyrPheAsnAspAlaLysGluGluSer


----.----+----.----+----.----+----.----+----.----+


GCGACAGCGAGGATTCTGTAACGTTCGAGTTCGTCCCTAACACCAAGAAG
----.----+----.----+----.----+----.----+----.----+    450
CGCTGTCGCTCCTAAGACATTGCAAGCTCAAGCAGGGATTGTGGTTCTTC

  AspSerGluAspSerValThrPheGluPheValProAsnThrLysLys


----.----+----.----+----.----+----.----+----.----+


CAAAAGTGCGGCAAGATCCGCATGCATGGGGTTCCGGGCGGCGGTGCTGG
----.----+----.----+----.----+----.----+----.----+    500
GTTTTCACGCCGTTCTAGGCGTACGTACCCCAAGGCCCGCCGCCACGACC

GlnLysCysGlyLysIleArgMetHisGlyValProGlyGlyGlyAlaGly


----.----+----.----+----.----+----.----+----.----+
```

```
CGGGGGTGGTGGACGAGACGTGAGCGGGGGCCCGAGCGACGGTCTGGGTG
----.----+----.----+----.----+----.----+----.----+   550
GCCCCCACCACCTGCTCTGCACTCGCCCCCGGGCTCGCTGCCAGACCCAC

  GlyGlyGlyGlyArgAspValSerGlyGlyProSerAspGlyLeuGlyGly


----.----+----.----+----.----+----.----+----.----+


GCGGTCGTGGTGGTGGGGGTGGTGGGGATTCCGGGGGGAATGATGGGGCGC
----.----+----.----+----.----+----.----+----.----+   600
CGCCAGCACCACCACCCCCACCACCCCTAAGGCCCCCTTACTACCCCGCG

   GlyArgGlyGlyGlyGlyGlyGlyAspSerGlyGlyMetMetGlyArg


----.----+----.----+----.----+----.----+----.----+


GGCGGTCGCATGTTGGGCGCTAGCGTGGACCGTACCTATCGGCTCAATTC
----.----+----.----+----.----+----.----+.----+   650
CCGCCAGCGTACAACCCGCGATCGCACCTGGCATGGATAGCCGAGTTAAG

GlyGlyArgMetLeuGlyAlaSerValAspArgThrTyrArgLeuAsnSer


----.----+----.----+----.----+----.----+----.----+


TAGAAAGATCCTGAAGAGCACGACGGGCATGAAAACGGTGGCTTTCGACC
----.----+----.----+----.----+----.----+.----+   700
ATCTTTCTAGGACTTCTCGTGCTGCCCGTACTTTTGCCACCGAAAGCTGG

  ArgLysIleLeuLysSerThrThrGlyMetLysThrValAlaPheAspLeu


----.----+----.----+----.----+----.----+----.----+


TATCGTCGCCCCAGAAGAGCGGTACGGGGCCGCAACCGGGTTCTGCCGGC
----.----+----.----+----.----+----.----+.----+   750
ATAGCAGCGGGGTCTTCTCGCCATGCCCCGGCGTTGGCCCAAGACGGCCG

   SerSerProGlnLysSerGlyThrGlyProGlnProGlySerAlaGly


----.----+----.----+----.----+----.----+----.----+
```

```
ATGGGGGGCGCCAAAACGCCGTCGGACGCCGTGCAGAACATCCTCCAAAA
----.----+----.----+----.----+----.----+----.----+   800
TACCCCCCGCGGTTTTGCGGCAGCCTGCGGCACGTCTTGTAGGAGGTTTT

MetGlyGlyAlaLysThrProSerAspAlaValGlnAsnIleLeuGlnLys


----.----+----.----+----.----+----.----+----.----+


GATCGAGAAGATTAAGAACACGGAGGAATAG
----.----+----.----+----.----+-   831
CTAGCTCTTCTAATTCTTGTGCCTCCTTATC

 IleGluLysIleLysAsnThrGluGluTer


----.----+----.----+----.----+-
```

Tab. 3: IgM-Reaktivität mit ausgesuchten Seren immunkompetenter Individuen mit akuter HCMV-Infektion, nachgewiesen durch Serokonversion bzw. durch Isolierung von CMV aus Blut oder Urin

| Patient Nr. | Referenztest IgM[1] | Referenzantigene | | GST-Fusion | | | Autol. Fusion | | UL57/3P |
|---|---|---|---|---|---|---|---|---|---|
| | | 150/7 | 52/3 | UL57/1 | UL57/2 | UL57/3 | 52/3 57/3 | 52/3 57/3 150/7/2 | |
| 1 | 5,7 | 1,576 | 0,353 | 0,014 | 0,030 | 3,0 | 1,155 | 0,993 | 0,863 |
| 2 | 6,0 | 2,019 | 2,166 | 0,057 | 0,147 | 3,0 | 3,0 | 3,0 | 3,0 |
| 3 | 1,0 | 3,0 | 0,533 | 0,085 | 0,179 | 0,896 | 0,417 | 1,733 | 0,058 |
| 4 | 7,4 | 3,0 | 0,971 | 0,036 | 0,092 | 0,546 | 1,110 | 3,0 | 0,090 |
| 5 | 7,4 | 2,473 | 2,305 | 0,392 | 0,486 | 3,0 | 2,387 | 2,096 | 0,413 |
| 6 | 5,2 | 0,692 | 0,382 | 0,007 | 0,016 | 1,413 | 0,896 | 0,578 | 0,407 |
| 7 | 5,3 | 0,411 | 0,077 | 0,043 | 0,039 | 3,0 | 1,704 | 0,941 | 0,688 |
| 8 | 3,8 | 0,764 | 0,533 | 0,020 | 0,037 | 0,658 | 0,732 | 0,783 | 0,138 |
| 9 | 4,2 | 1,268 | 0,249 | 0,053 | 0,042 | 3,0 | 0,592 | 0,413 | 0,325 |
| 10 | 5,8 | 1,470 | 1,413 | 0,086 | 0,198 | 3,0 | 2,626 | 2,380 | 2,049 |
| 11a 14.8.92 | 2,0 | 3,0 | 0,545 | 0,016 | 0,077 | 3,0 | 0,897 | 0,831 | 0,714 |
| 11b 1.9.92 | 6,0 | 3,0 | 2,019 | 0,061 | 0,118 | 3,0 | 3,0 | 2,700 | 2,807 |
| 12 | 2,1 | 0,289 | 1,863 | 0,052 | 0,231 | 3,0 | 3,0 | 2,778 | 2,818 |
| 13 | n.d. | 2,188 | 1,740 | 0,082 | 0,207 | 1,740 | 2,245 | 2,360 | 1,444 |
| Σ 14 | über cut-off: | 13 | 12 | 1 | 1 | 14 | 14 | 14 | 11 |

[1]sample/cut-off ratio (Medac IgM)

▦ = Ergebnisse über dem vorläufig festgelegten cut-off von OD 0,3

Abb. 11

EP 0 702 083 A2

Tab.4.: IgM-Reaktivität mit ausgesuchten Seren von Transplantationspatienten mit akuter HCMV-Primärinfektion
Die Seren wurden 2 - 3 Wochen nach Beginn der Antigenemie entnommen.

| Patient Nr. | pos. Zellen/ 50000 Antigenemie | Referenzantigene | | GST-Fusion | | | Autol. Fusion | | |
|---|---|---|---|---|---|---|---|---|---|
| | | 150/7 | 52/3 | UL57/1 | UL57/2 | UL57/3 | 52/3 57/3 | 52/3 57/3 150/7/2 | UL52/3P |
| 1 | 13 | 1,996 | 0,771 | 0,024 | 0,064 | 3,0 | 1,535 | 1,750 | 1,394 |
| 2 | 113 | 0,220 | 0,191 | 0,103 | 0,031 | 0,273 | 0,171 | 0,188 | 0,030 |
| 3 | 15 | 2,648 | 1,186 | 0,021 | 0,037 | 2,051 | 1,397 | 1,458 | 0,925 |
| 4 | 30 | 1,660 | 0,878 | 0,127 | 0,234 | 1,034 | 0,821 | 0,891 | 0,418 |
| 5 | 0 | 2,761 | 1,936 | 0,040 | 0,149 | 3,0 | 3,0 | 3,0 | 3,0 |
| 6 | 135 | 1,768 | 0,993 | 0,813 | 0,123 | 2,697 | 2,742 | 2,352 | 2,662 |
| 7 | 25 | 2,705 | 2,259 | 0,679 | 0,271 | 3,0 | 3,0 | 2,830 | 3,0 |
| 8 | 7 | 0,987 | 0,806 | 0,038 | 0,071 | 2,773 | 1,879 | 0,951 | 1,088 |
| 9 | 40 | 3,0 | 0,404 | 0,069 | 0,087 | 0,980 | 0,688 | 1,803 | 0,135 |
| Σ 9 | über cut-off: | 8 | 8 | 2 | — | 8 | 8 | 8 | 7 |

= Ergebnisse über dem vorläufig festgelegten cut-off von OD 0,3

Abb. 12

EP 0 702 083 A2

**Tab. 5: IgM-Reaktivität der rekombinanten Antigene mit Seren gesunder Blutspender**

| | 150/7 | 52/3 | UL57/1-GST | UL57/2-GST | UL57/3-GST | 52/3 57/3 | 52/3 57/3 150/7/2 | UL57/3P |
|---|---|---|---|---|---|---|---|---|
| **A. Blutspender CMV-seropositiv** n = 54 | 19 | 1 | — | — | 1 | 1 | 2 | 1 |
| **B. Blutspender CMV-seronegativ** n = 54 | — | — | — | — | 1 | — | — | — |

Abb. 13

EP 0 702 083 A2

**Tab. 6 : Kurze Charakterisierung der evaluierten Antigene**

| Antigen | Charakterisierung | Leserahmen Aminosäurebereich | Diagnostische Bedeutung |
|---|---|---|---|
| UL57/1-GST | GST-Fusionsprotein | UL57 aa 755-1000 | — |
| UL57/2-GST | GST-Fusionsprotein | UL57 aa 1144-1196 | — |
| UL57/3-GST | GST-Fusionsprotein | UL57 aa 545-601 | + + + |
| UL57/3-P | Peptid | UL57 aa 545-601 | + + |
| 52/3 57/3 | autologes Fusionsprotein | UL44 aa 297-433<br>+ UL57 aa 545-601 | + + + |
| 52/3 57/3 150/7/2 | autologes Fusionsprotein | UL44 aa 297-433<br>+ UL57 aa 545-601<br>+ UL32 aa 994-1048 | + + + |

Abb. 14

EP 0 702 083 A2